(19) Europäisches Patentamt
European Patent Office
Office européen des brevets

(11) Publication number: 0 055 418
B1

(12) EUROPEAN PATENT SPECIFICATION

(45) Date of publication of patent specification: 26.02.86

(21) Application number: 81110457.9

(22) Date of filing: 15.12.81

(51) Int. Cl.[4]: C 07 D 231/06, C 07 D 401/04, A 61 K 31/415, A 61 K 31/44, A 61 K 31/47 // (C07D401/04, 231:06, 213:76),(C07D401/04, 231:06, 215:38)

# (54) Pyrazoline derivatives, processes for their preparation and pharmaceutical formulations containing them.

(30) Priority: 16.12.80 GB 8040172
21.04.81 US 256072

(43) Date of publication of application:
07.07.82 Bulletin 82/27

(45) Publication of the grant of the patent:
26.02.86 Bulletin 86/09

(84) Designated Contracting States:
BE CH DE FR GB IT LI LU NL SE

(56) References cited:
EP-A-0 022 578
GB-A-1 297 035

The file contains technical information submitted after the application was filed and not included in this specification

(73) Proprietor: THE WELLCOME FOUNDATION LIMITED
183-193 Euston Road
London NW1 2BP (GB)

(72) Inventor: Copp, Frederick Charles
"Rotherwood" 32 Stanley Avenue
Beckenham Kent (GB)
Inventor: Caldwell, Albert Gordon
119 Gates Green Road
West Wickham Kent (GB)
Inventor: Collard, David
25 The Mead
Beckenham Kent (GB)

(74) Representative: Sandmair, Kurt, Dr. et al
Patentanwälte Schwabe, Sandmair, Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 418 B1

**Description**

This invention relates to pharmaceutical formulations comprising heterocyclic compounds, i.e. 1-aryl-3-(substituted)amino-2-pyrazolines and their preparation, to their use in medicine in a mammal, including man, e.g. as anti-inflammatory or anti-allergic agents or as agents in the prevention of tissue rejection, and to certain novel heterocyclic compounds and their preparation.

In GB—A—1297035 5-aryl-3-amino-5-alkyl-2-pyrazolines are disclosed wherein the aryl group is attached to a ring carbon atom and not to a ring nitrogen atom as in the present invention.

In their studies on the reaction of diazonium salts with 1-aryl-2-pyrazolines, Duffin and Kendall (J. Chem. Soc., (1954), 408—415) produced 3-ethylamino-1-phenyl-2-pyrazoline (pages 409 and 413) in tests to identify the product of an earlier reaction. It has now been found that related compounds of formula (I) inhibit both the lipoxygenase and cyclo-oxygenase pathways of arachidonic acid metabolism in vitro and are useful as anti-inflammatory or anti-allergic agents or as agents in the prevention of tissue rejection and other medical conditions alleviated by the inhibition of arachidonic acid oxygenation.

In EP—A—0 022 578 compounds are disclosed structurally similar to those of the present invention namely 1-aryl-3-(unsubstituted)amino-2-pyrazolines.

Accordingly, the present invention relates to pharmaceutical compositions containing heterocyclic compounds of formula (I) and acid addition salts thereof:

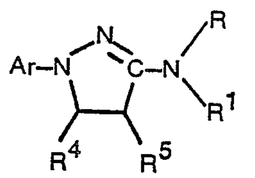

(I)

wherein

Ar is a group selected from phenyl, naphthyl, quinolyl and pyridyl, which group may be optionally substituted by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms;

R is selected from hydrogen, $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl;

$R^1$ is selected from $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl;

Certain of these compounds are novel, namely the compounds defined in claim 12.

The compounds of formula (I) exhibit many advantages in their anti-inflammatory (and related) activities over hitherto-known anti-inflammatory compounds. Since they inhibit the cyclo-oxygenase pathway of arachidonic acid metabolism, the compounds of formula (I) exhibit some of the properties of the non-steroidal anti-inflammatory (or, aspirin-like) drugs, such as: reduction of erythema, oedema and pain production associated with prostaglandins leading to good symptomatic relief. However, aspirin-like drugs are not effective against the leukocyte-mediated aspects of chronic inflammatory diseases.

Anti-inflammtory corticosteroids indirectly inhibit the production of both prostaglandins and leukotrienes which could, in part, account for their superior therapeutic effects. However, steroid treatment is associated with serious local or systemic side-effects and their prolonged administration is contraindicated in many inflammatory conditions.

The present invention therefore fulfills the need for a non-steroid compound which inhibits the synthesis of leukotrienes and prostaglandins, and which is free from steroid-related toxicity. In addition, it provides compounds which, whilst inhibiting both pathways of arachidonic acid metabolism, exhibit selectivity in inhibiting the lipoxygenase pathway more than the cyclo-oxygenase pathway (see Table I). Furthermore, it provides compounds which are more chemically stable and compounds which exhibit a broader therapeutic range than related anti-inflammatories.

The present invention will now be described in more detail with reference to formula (I) above.

In this specification "alkyl", "alkenyl", and "alkynyl" groups each have from 1 to 6 carbon atoms.

In this specification Ar is an "aromatic" radicals selected from phenyl, naphthyl, quinolyl and pyridyl. Particularly preferred aromatic radicals are phenyl and pyridyl, especially wherein 'pyridyl' is selected from

2-pyridyl and 4-pyridyl. The aromatic ring is preferably substituted and the substituents are selected from halo, alkyl (which may itself be optionally substituted by halo), carboxy, alkoxy, amino which may itself be optionally substituted by 1 or 2 alkyl groups, hydroxy and alkylsulphonyl of which the alkyl moiety may itself be optionally substituted by halo. Examples of especially suitable Ar substituents are halo (that is: fluoro, chloro, bromo and iodo), *tert*-butyl and trifluoromethyl. When Ar is phenyl, the preferred positions of the ring for any substituent are those selected from the 2-, 3-, 4-, 3,4- and 2,6- positions. For example, Ar may be selected from 3-trifluoromethylphenyl, 4-trifluoromethylphenyl, 4-fluorophenyl, 4-chlorophenyl, 4-bromophenyl, 3-trifluoromethyl-4-fluorophenyl, 3-trifluoromethyl-4-chlorophenyl and 3-trifluoromethyl-4-bromophenyl. When Ar is pyridyl, the preferred positions of the ring for any substituent are those selected from the 5- and 6- positions. For example, Ar may be selected from 5-chloro-2-pyridyl, 5-bromo-2-pyridyl and 5-iodo-2-pyridyl.

$R^4$ and $R^5$ are preferably selected from hydrogen and methyl. $R^1$ is preferably selected from alkyl and benzyl. R is preferably selected from hydrogen, alkyl and benzyl and more preferably from hydrogen and alkyl. For example, compounds of formula (I) when R is hydrogen and $R^1$ is alkyl such as methyl are especially preferred. R or $R^1$ may also be 1-phenylethyl or benzyl substituted by a substituent listed above in the examples of 'aromatic radicals', preferably chloro or methoxy.

Examples of compounds of formula (I) are:

3-(methylamino)-1-(3-trifluoromethylphenyl)-2-pyrazoline;
3-(ethylamino)-1-(3-trifluoromethylphenyl)-2-pyrazoline;
3-(*n*-propylamino)-1-(3-trifluoromethylphenyl)-2-pyrazoline;
3-(*iso*-propylamino)-1-(3-trifluoromethylphenyl)-2-pyrazoline;
3-(*n*-butylamino)-1-(3-trifluoromethylphenyl)-2-pyrazoline;
3-(*sec*-butylamino)-1-(3-trifluoromethylphenyl)-2-pyrazoline;
3-(*tert*-butylamino)-1-(3-trifluoromethylphenyl)-2-pyrazoline;
3-(benzylamino)-1-(3-trifluoromethylphenyl)-2-pyrazoline;
3-(*N,N*-dimethylamino)-1-(4-trifluoromethylphenyl)-2-pyrazoline;
3-(*N*-methyl-*N*-ethylamino)-1-(4-chlorophenyl)-2-pyrazoline;
3-(methylamino)-1-(4-fluorophenyl)-5-methyl-2-pyrazoline;
3-(benzylamino)-1-(4-bromophenyl)-4-methyl-2-pyrazoline;
3-(ethylamino)-1-(3-trifluoromethyl-4-fluorophenyl)-2-pyrazoline;
3-(*tert*-butylamino)-1-(3-trifluoromethyl-4-bromophenyl)-2-pyrazoline;
3-(*iso*-propylamino)-1-(5-chloro-2-pyridyl)-2-pyrazoline;
3-(methylamino)-1-(5-bromo-2-pyridyl)-2-pyrazoline;
3-(benzylamino)-1-(5-iodo-2-pyridyl)-2-pyrazoline;
3-(allylamino)-1-(3-trifluoromethylphenyl)-2-pyrazoline;
3-(cyclohexylmethylamino)-1-(3-trifluoromethylphenyl)-2-pyrazoline;
3-methylamino-1-(5-methyl-2-pyridyl)-2-pyrazoline;
3-methylamino-1-(6-methyl-2-pyridyl)-2-pyrazoline;
3-ethylamino-1-(6-methyl-2-pyridyl)-2-pyrazoline;
3-methylamino-1-(3-quinolyl)-2-pyrazoline;
3-methylamino-1-(4-chloro-3-trifluoromethylphenyl)-2-pyrazoline;
3-methylamino-1-(5-bromo-6-methyl-2-pyridyl)-2-pyrazoline;
3-(N,N-dimethylamino)-1-(3-trifluoromethylphenyl)-2-pyrazoline;
3-ethylamino-5-methyl-1-phenyl-2-pyrazoline;
3-butylamino-5-methyl-1-phenyl-2-pyrazoline;
3-(1-phenylethylamino)-1-(3-trifluoromethylphenyl)-2-pyrazoline;
3-(2-butenylamino)-1-(2-naphthyl)-2-pyrazoline;
3-ethylamino-4-methyl-1-(3-trifluoromethylphenyl)-2-pyrazoline;
3-benzylamino-1-(3-t-butylphenyl)-2-pyrazoline;
3-benzylamino-1-(3-carboxyphenyl)-2-pyrazoline;
3-methylamino-1-(2-chlorophenyl)-2-pyrazoline;
3-benzylamino-1-(4-methoxyphenyl)-2-pyrazoline;
3-(4-chlorobenzylamino)-1-(4-chlorophenyl)-2-pyrazoline; and
3-(4-methoxybenzylamino)-1-(4-bromo-3-trifluoromethylphenyl)-2-pyrazoline.

A novel subclass of the compounds of formula (I) is the compounds of formula (IA):

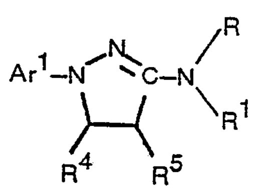

(IA)

wherein

$Ar^1$ is selected from pyridyl, quinolyl and naphthyl optionally substituted in any position of the ring by one or more substituent(s) as defined in Formula (I);

R is selected from hydrogen, $C_{1-6}$ alkyl (optionally substituted by a substituent selected from phenyl which may itself be substituted as defined in formula (I) and cycloalkyl of from 3 to 6 carbon atoms), $C_{2-6}$ alkenyl and $C_{2-6}$ alkynyl;

$R^1$ is selected from $C_{1-6}$ alkyl (optionally substituted by a substituent selected from phenyl which may itself be substituted as defined in formula (I) and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl; and acid salts thereof.

A further novel subclass of the compounds of formula (I) is the compounds of formula (IB):

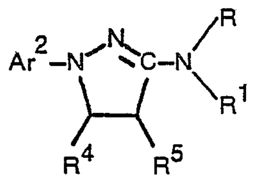

(IB)

wherein

$Ar^2$ is phenyl substituted in any position of the ring by one or more substituent(s) as defined in Formula (I);

R is selected from hydrogen, $C_{1-6}$ alkyl (optionally substituted by a substituent selected from phenyl which may itself be substituted as defined in formula (I) and cycloalkyl of from 3 to 6 carbon atoms), $C_{2-6}$ alkenyl and $C_{2-6}$ alkynyl;

$R^1$ is selected from $C_{1-6}$ alkyl (optionally substituted by a substituent selected from phenyl which may itself be substituted as defined in formula (I) and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl; and acid addition salts thereof.

A still further novel subclass of the compounds of formula (I) is the compounds of formula (IC):

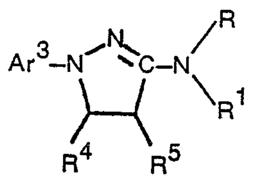

(IC)

wherein

$Ar^3$ is an unsubstituted phenyl group;

R is selected from $C_{1-6}$ alkyl (substituted by a substituent selected from phenyl which may itself be substituted as defined in formula (I) and cycloalkyl of from 3 to 6 carbon atoms), $C_{2-6}$ alkenyl and $C_{2-6}$ alkynyl;

$R^1$ is selected from $C_{1-6}$ alkyl (substituted by a substituent selected from phenyl which may itself be substituted as defined in formula (I) and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl; and acid addition salts thereof.

Preferred compounds of formula (IA), (IB) or (IC) are those wherein R and $R^1$ are each other than alkyl (substituted by substituted-phenyl); such compounds are here defined as falling within formula (IE), (IF) or (IG), respectively.

However, a preferred subclass of the compounds of formula (I), within the subclasses of formulae (IA) or (IE) and (IB) or (IF) described above, is the compounds of formula (ID):

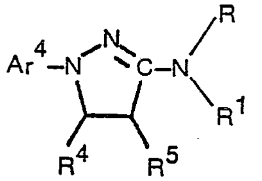

(ID)

wherein

$Ar^4$ is a monocyclic aromatic radical selected from pyridyl which is optionally substituted in any position of the ring by one or more substituent(s) as defined in Formula (I); and phenyl substituted in any position of the ring by one or more substituent(s);

R is selected from hydrogen and $C_{1-6}$ alkyl (optionally substituted by phenyl);

$R^1$ is $C_{1-6}$ alkyl (optionally substituted by phenyl); and

$R^4$ and $R^5$ are the same or different and are each selected from hydrogen and $C_{1-6}$ alkyl; and acid addition salts thereof.

When used in medicine, the acid addition salts of a compound of formula (I) should be both pharmacologically and pharmaceutically acceptable acid addition salts, but non-acceptable salts may conveniently be used to prepare the bases of such acceptable salts and are not excluded from the scope of this invention. Acceptable salts may be derived from organic acids, particularly dicarboxylic acids. Such pharmacologically and pharmaceutically acceptable salts include those prepared from the following acids: hydrochloric, hydrobromic, sulphuric, nitric, phosphoric, oxalic, fumaric, maleic, glycolic, salicylic, succinic, toluene-*p*-sulphonic, tartaric, acetic, citric, methane sulphonic, formic, benzoic, malonic, naphthalene-2-sulphonic and benzene sulphonic.

The compounds of formula (I) may be prepared by any method analogous to those known in the art, for example by the method of G. F. Duffin and J. D. Kendall in J. Chem. Soc. (1954), 408—415. Other methods include:

(1) A method for preparing a compound of formula (I) comprises cyclisation and elimination of water from a compound of formula (II) and optionally converting it to any other desired compound of formula (I).

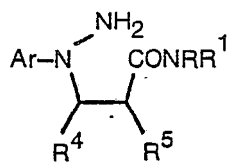
(II)

wherein R, $R^1$, $R^4$, $R^5$ and Ar are as defined in formula (I). Suitable agents include phosphorous oxychloride ($POCl_3$).

The compound of the formula (II) may itself be prepared by reaction of the corresponding compound of formula (III) with the corresponding compound of formula (IV).

$Ar—NH—NH_2$ (III)

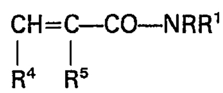
(IV)

where Ar, R, $R^1$, $R^4$ and $R^5$ are as defined in formula (II).

A preferred reaction is where either R is other than hydrogen or, when R is hydrogen, R is sterically hindered by $R^1$ which is therefore a group such as *tert*-butyl.

(2) A further method, wherein, in formula (I), R is H and $R^1$ is other than a group having a terminal acetylenic hydrogen atom, comprises reaction of a compound of formula (VII) with $R^8Y$:

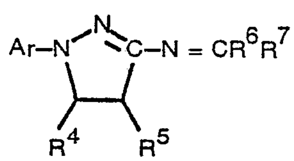
(VII)

wherein Ar, $R^4$ and $R^5$ are as defined in formula (I); Y is an alkali metal or an alkaline earth metal halide; and $—CR^6R^7R^8$ is $R^1$ as defined in formula (I). For example, $R^8Y$ is an alkyl alkaline earth metal halide such as a Grignard agent for example methylmagnesium iodide; or an alkyl alkali metal such as butyl lithium. For example, a method of preparing a compound of formula (I) wherein $R^1$ is *iso*propyl comprises reaction of a compound of formula (VII) wherein $R^6$ is H and $R^7$ is methyl with methyl magnesium iodide. Preferably, Ar does not include a carboxy group.

(3) A further method wherein R is hydrogen comprises reduction of a compound of formula (VII) wherein Ar, $R^4$ and $R^5$ are as defined in formula (I); and $—CHR^6R^7$ is $R^1$ as defined in formula (I). Suitable reducing agents are known to those skilled in the art and include sodium borohydride or another metallic reducing agent such as sodium cyano-borohydride; or, where $R^1$ does not include a —C=C— or a —C=C—, by catalytic reduction using, for example, a catalyst such as platinum or palladium on carbon; or, where Ar includes halo, by non-catalytic agents such as sodium borohydride.

(4) A further method, wherein, in formula (I), R is hydrogen and $R^1$ includes a methylene adjacent the nitrogen atom and preferably does not include alkynyl, comprises reduction of a compound of formula (X).

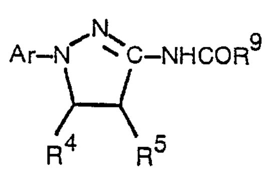
(X)

wherein Ar, $R^4$ and $R^5$ are as defined in formula (I) and either $CH_2R^9$ is $R^1$ as defined in formula (I), (for example, if $R^1$ is methyl, $R^9$ is hydrogen or if $R^1$ is ethyl, $R^9$ is methyl) or, when $R^1$ is methyl, $R^9$ may be alkoxy, for example ethoxy. Suitable reducing agents are known to those skilled in the art and include diborane and lithium aluminium hydride.

The compounds of formula (I) may be used in the relief of rheumatoid arthritis, rheumatoid spondylitis, osteoarthritis, gouty arthritis and other arthritic conditions, inflamed joint, eczema, other inflammatory skin conditions, inflammatory eye conditions including conjunctivitis, pyresis and other conditions associated with inflammation and pain. Such other conditions associated with inflammation include the reduction of tissue necrosis in chronic inflammation, the suppression of tissue rejection following transplant surgery and ulcerative colitis.

The compounds of formula (I) may also be used in the treatment or prophylaxis of allergic conditions and other airway inflammatory conditions such as asthma and of asthma having a non-allergic origin and bronchitis. The compounds may also be useful as antispasmogenic agents.

The amount required of a compound of formula (I) (hereinafter referred to as the active ingredient) for therapeutic effect will, of course, vary both with the particular compound, the route of administration and the mammal under treatment. A suitable dose of a compound of formula (I) for a mammal suffering from an inflammatory, painful or pyretic condition as defined hereinbefore is 0.5 to 500 mg of base of per kilogram bodyweight, the most preferred dosage being 0.5 to 50 mg/kg of mammal bodyweight for example 5 to 25 mg/kg; administered two or three times daily.

In the case of the treatment or prophylaxis of inflammatory airway conditions, a suitable anti-asthmatic dose of a compound of formula (I) is 1 mg to 10 mg of base per kilogram bodyweight, the most preferred dosage being 1 mg to 5 mg/kg of mammal bodyweight, for example from 1 to 2 mg/kg.

While it is possible for an active ingredient to be administered alone as the raw chemical, it is preferable to present it as a pharmaceutical formulation. Conveniently, the active ingredient comprises from 0.1% to 99.9% by weight of the formulation. Conveniently, unit doses of a formulation contain between 0.1 mg and 1 g of the active ingredient. For topical administration, the active ingredient preferably comprises from 1% to 2% by weight of the formulation but the active ingredient may comprise as much as 10% w/w. Formulations suitable for nasal or buccal administration, (such self-propelling powder-dispensing formulations described hereinafter), may comprise 0.1 to 20% w/w, for example about 2% w/w of active ingredient.

The formulations, both for veterinary and for human medical use, of the present invention comprise an active ingredient in association with a pharmaceutically acceptable carrier therefor and optionally other therapeutic ingredient(s). The carrier(s) must be 'acceptable' in the sense of being compatible with the other ingredients of the formulations and not deleterious to the recipient thereof.

The formulations include those in a form suitable for oral, ophthalmic, rectal, parenteral (including subcutaneous, intramuscular and intravenous), intra-articular, topical, nasal or buccal administration.

The formulations may conveniently be presented in unit dosage form and may be prepared by any of the methods well known in the art of pharmacy. All methods include the step of bringing the active ingredient into association with the carrier which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units such as capsules, cachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be in the form of a bolus, electuary or paste.

A tablet may be made by compressing or moulding the active ingredient optionally with one or more accessory ingredients. Compressed tablets may be prepared by compressing, in a suitable machine, the active ingredient in a free-flowing form such as a powder or granules, optionally mixed with a binder, lubricant, inert diluent, surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration conveniently comprise a sterile aqueous preparation of the active ingredient which is preferably isotonic with the blood of the recipient.

Formulations suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical administration include liquid or semi-liquid preparations such as liniments, lotions, applications; oil-in-water or water-in-oil emulsions such as creams, ointments or pastes; or solutions or suspensions such as drops. For example, for ophthalmic administration, the active ingredient may be presented in the form of aqueous eye drops as, for example, a 0.1—1.0% solution.

Formulations suitable for administration to the nose or buccal cavity include powder, self-propelling and spray formulations such as aerosols and atomizers. The formulations, when dispersed, preferably have a particle size in the range of 10 to 200 μ.

Such formulations are most preferably in the form of a finely comminuted powder for pulmonary administration from a powder inhalation device or self-propelling powder-dispensing formulations, where the active ingredient, as a finely comminuted powder, may comprise up to 99.9% w/w of the formulation. In the case of self-propelling solution and spray formulations, the effect may be achieved either by choice of a valve having the desired spray characteristics (i.e. being capable of producing a spray having the desired particle size) or by incorporating the active ingredient as a suspended powder if controlled particle size. Thus the formulation, instead of passing into the lungs, is largely retained in the nasal cavity. These self-propelling formulations may be either powder-dispensing formulations or formulations dispensing the active ingredient as droplets of a solution or suspension.

Self-propelling powder-dispensing formulations preferably comprise dispersed particles of solid active ingredient, and a liquid propellant having a boiling point if below 18°C at atmospheric pressure. The liquid propellant may be any propellant known to be suitable for medicinal administration and may comprise one or more lower alkyl hydrocarbons or halogenated lower alkyl hydrocarbons or mixtures thereof; chlorinated and fluorinated lower alkyl hydrocarbons are especially preferred. Generally, the propellant constitutes 50 to 99.9% w/w of the formulation whilst the active ingredient constitutes 0.1 to 20% w/w, for example, about 2% w/w, of the formulation.

The pharmaceutically acceptable carrier in such self-propelling formulations may include other constituents in addition to the propellant, in particular a surfactant or a solid diluent or both. Surfactants are desirable since they prevent agglomeration of the particles of active ingredient and maintain the active ingredient in suspension. Especially valuable are liquid non-ionic surfactants and solid anionic surfactants or mixtures thereof. Suitable liquid non-ionic surfactants are those having a hydrophile-lipophile balance (HLB, see Journal of the Society of Cosmetic Chemists Vol. 1 pp. 311—326 (1949)) of below 10, in particular esters and partial esters of fatty acids with aliphatic polyhydric alcohols, for instance, sorbitan monooleate and sorbitan trioleate, known commercially as 'Span 80' (Trade Name) and 'Span 85' (Trade Name), respectively. The liquid non-ionic surfactant may constitute from 0.01 up to 20% w/w of the formulation, though preferably it constitutes below 1% w/w of the formulation. Suitable solid anionic surfactants include alkali metal, ammonium and amine salts of dialkyl sulphosuccinate (where the alkyl groups have 4 to 12 carbon atoms) and alkyl benzene sulphonic acid (where the alkyl group has 8 to 14 carbon atoms). The solid anionic surfactants may constitute from 0.01 up to 20% w/w of the formulation, though preferably below 1% w/w of the composition solid diluents may be advantageously incorporated in such self-propelling formulations where the density of the active ingredient differs substantially from the density of the propellant; also, they help to maintain the active ingredient in suspension. The solid diluent is in the form of a fine powder, preferably having a particle size of the same order as that of the particles of the active ingredient. Suitable solid diluents include sodium chloride, sodium sulphate and sugars.

Formulations of the present invention may also be in the form of a self-propelling formulation wherein the active ingredient is present in solution. Such self-propelling formulations may comprise the active ingredient, propellant and co-solvent, and advantageously an antioxidant stabiliser. The propellant is one or more of these already cited above. Co-solvents are chosen for their solubility in the propellant, their ability to dissolve the active ingredient, and for their having the lowest boiling point consistent with these above-mentioned properties. Suitable co-solvents are lower alkyl alcohols and ethers and mixtures thereof. The co-solvent may constitute 5 to 40% w/w of the formulation, though preferably less than 20% w/w of the formulation. Antioxidant stabilisers may be incorporated in such solution-formulations to inhibit deterioration of the active ingredient and are conveniently alkali metal ascorbates or bisulphites. They are preferably present in an amount of up to 0.25% w/w of the formulation.

Such self-propelling formulations may be prepared by any method known in the art. For example, the active ingredient (either as particles as defined hereinbefore in suspension in a suitable liquid or in up to 20% w/v solution in an acceptable co-solvent, as appropriate) is mixed with any other constituents of a pharmaceutically acceptable carrier. The resulting mixture is cooled, introduced into a suitable cooled container and propellant is added thereto in liquid form; and the container is sealed. Alternatively, such self-propelling formulations may be prepared by mixing the active ingredient either in particles as hereinbefore defined or in 2 to 20% w/v alcohol or aqueous solution as appropriate, together with the remaining constituents of the pharmaceutically acceptable carrier other than the propellant; introducing the resulting mixture, optionally with some propellant, into a suitable container; and injecting the propellant, under pressure, into the container at ambient temperature through a valve which comprises a part of the container and is used to control release of the formulation from it. Desirably, the container is purged by removing air from it at a convenient stage in the preparation of the self-propelling formulation.

A suitable container for a self-propelling formulation is one provided with a manually-operable valve and constructed of aluminium, stainless steel or reinforced glass. The valve should, of course, be one having the desired spray characteristics of particle size as hereinbefore defined. Advantageously, the valve is of the type which delivers a fixed amount of the formulation on the occasion of each operation of the valve, for example, about 50 to 100 microlitres of formulation in each delivery.

Formulations of the present invention may also be in the form of an aqueous or dilute alcoholic

solution, optionally a sterile solution, of the active ingredient for use in a nebuliser or atomiser, wherein an accelerated air stream is used to produce a fine mist consisting of small droplets of the solution. Such formulations usually contain a flavouring agent such as saccharin sodium and a volatile oil. A buffering agent such as sodium metabisulphite and a surface active agent may also be included in such a formulation which should also contain a preservative such as methylhydroxybenzoate.

Other formulations suitable for nasal administration include a coarse powder having a particle size of 20 to 500 microns which is administered in the manner of which snuff is taken i.e. by rapid inhalation through the nasal passage from a container of the powder held close up to the nose.

In addition to the aforementioned ingredients, the formulations of this invention may include one or more additional ingredients such as diluents, buffers, flavouring agents, binder, surface active agents, thickeners, lubricants, preservatives eg. methylhydroxybenzoate (including anti-oxidants), emulsifying agents and the like.

Any other therapeutic ingredient may comprise one or more of the following: anti-biotic, anti-fungal and anti-viral agents.

According to the present invention there are therefore provided:—

(a) a novel compound of formula (I) or an acid addition salt thereof;

(b) a method for preparing a compound of formula (I);

(c) a pharmaceutical formulation comprising a non-toxic, effective arachidonic acid oxygenation inhibitory amount of a compound of formula (I) or a pharmaceutically acceptable salt thereof and a pharmaceutically acceptable carrier therefor;

(d) a method for preparing such formulations;

(e) a compound of formula (I) or an acid addition salt thereof for use in a method for the prophylaxis or treatment of inflammation in a mammal, including man, comprising the administration to said mammal of a non-toxic, effective anti-inflammatory amount of a compound of formula (I);

(f) a compound of formula (I) or an acid addition salt thereof for use in a method for the prophylaxis or treatment of pain in a mammal, including man, comprising the administration to said mammal of a non-toxic, effective analgesic amount of a compound of formula (I);

(g) a compound of formula (I) or an acid addition salt thereof for use in a method for the prophylaxis or treatment of pyresis in a mammal, including man, comprising the administration to said mammal of a non-toxic, effective anti-pyretic amount of a compound of formula (I);

(h) a compound of formula (I) or an acid addition salt thereof for use in a method for the prophylaxis or treatment of asthma in a mammal, including man, comprising administration to said mammal of a non-toxic, effective, anti-asthmatic amount of a compound of formula (I);

(i) a compound of formula (I) or an acid addition salt thereof for use in a method for the inhibition of the lipoxygenase or cyclo-oxygenase pathways of arachidonic acid metabolism comprising the administration of a non-toxic, effective, inhibitory amount of a compound of formula (I) or a pharmaceutically acceptable acid addition salt thereof; and

(j) a compound of formula (I) for use in medicine in the inhibition of the lipoxygenase or cyclo-oxygenase pathways of arachidonic acid metabolism.

The following Examples are provided by way of an illustration of the present invention and should in no way be construed as a limitation thereof. All temperatures indicated are in degrees Celsius.

Example 1

Preparation of 3-methylamino-1-(3-trifluoromethylphenyl)-2-pyrazoline

3-Formamido-1-(3-trifluoromethylphenyl)-2-pyrazoline (2.4 g) was stirred with dry diethyl ether (60 ml) to produce a slurry which was gradually added to a stirred ice-cold suspension of lithium aluminium hydride (1.0 g) in dry diethyl ether (75 ml) under a nitrogen atmosphere. The addition took place over a period of about 20 minutes and was accompanied by a vigorous reaction. The final reaction mixture was stirred for a further 15 minutes and was then carefully and slowly decomposed by the addition of water (30 ml). The ethereal layer was then decanted off the aqueous sludge which was washed twice with diethyl ether. The combined ethereal solutions were dried over potassium carbonate, filtered and evaporated. The resulting 3-methylamino-1-(3-trifluoromethyl)-2-pyrazoline was obtained as a colorless crystalline solid on trituration with benzene and recrystallization from light petroleum, yield 1.8 g, m.p. 93.1°. The hydrochloride was recrystallized by precipitation from methanol with ether and light petroleum m.p. 206—207°.

Example 2

Preparation of 3-ethylamino-1-(3-trifluoromethylphenyl)-2-pyrazoline

By a method analogous to that described in Example 1 3-acetamido-1-(3-trifluoromethylphenyl)-2-pyrazoline was reduced with lithium aluminium hydride, using tetrahydrofuran in place of diethyl ether. The hydrochloride of the resulting 3-ethylamino-1-(3-trifluoromethylphenyl)-2-pyrazoline was recrystallized from isopropanol and diethylether, m.p. 171.5°.

Example 3

Preparation of 3-propylamino-1-(3-trifluoromethylphenyl)-2-pyrazoline

3-Propionamido-1-(3-trifluoromethylphenyl)-2-pyrazoline was prepared from propionic anhydride and

3-amino-1-(3-trifluoromethylphenyl)-2-pyrazoline in refluxing chloroform and crystallized from methanol m.p. 175.2°. It was then reduced with lithium aluminium hydride in diethyl ether according to the method described in Example 1 to give 3-propylamino-1-(3-trifluoromethylphenyl)-2-pyrazoline which was recrystallized as the hydrochloride m.p. 151.2°.

Example 4

Preparation of 3-butylamino-1-(3-trifluoromethylphenyl)-2-pyrazoline

According to the method of Example 1 3-butyramido-1-(3-trifluoromethylphenyl)-2-pyrazoline was reacted with lithium aluminium hydride to produce 3-butylamino-1-(3-trifluoromethylphenyl)-2-pyrazoline which was a gum (yield 2.4 g) and was recrystallized as the hydrochloride m.p. 173.4° (yield 1.65 g).

Example 5

Preparation of 3-benzylamino-1-(3-trifluoromethylphenyl)-2-pyrazoline hydrochloride

3-Amino-1-(3-trifluoromethylphenyl)-2-pyrazoline (460 mg) and benzaldehyde (220 mg) were dissolved together in ethanol (2 ml) and the solution was heated to reflux for 2 hours. On cooling, the separated 3-benzylideneamino-1-(3-trifluoromethylphenyl)-2-pyrazoline was collected and recrystallized from ethanol, m.p. 159—160°.

3-Benzylideneamino-1-(3-trifluoromethylphenyl)-2-pyrazoline (1.6 g) was suspended in ethanol (10 ml) at room temperature under a nitrogen atmosphere. The mixture was stirred and sodium borohydride (2.0 g) was added. After stirring for some 2 hours, a clear light-yellow solution formed, which was poured onto ice and diethyl ether. The aqueous portion was removed, and the residual diethyl ether was washed with fresh water and then with 2*N*-hydrochloric acid. A crystalline solid separated which was collected, washed with fresh diethyl ether and then with fresh 2*N*-hydrochloric acid. The resulting 3-benzylamino-1-(3-trifluoromethylphenyl)-2-pyrazoline hydrochloride was recrystallized from methanol, diethyl ether and light petroleum, m.p. 190.3° (yield 890 mg).

Example 6

Preparation of starting material of Example 1

3-Amino-1-(3-trifluoromethylphenyl)-2-pyrazoline (1.907 g) was dissolved in formic acid (20 ml). The solution was stirred at 60° and acetic anhydride (2.4 ml) was added dropwise. The mixture was heated for 1 hour at 60° and then poured into water and stirred to decompose the residual excess anhydride. 3-Formamido-1-(3-trifluoromethylphenyl)-2-pyrazoline resulted as a crystalline solid m.p. 128—129°. This compound was then used to prepare the compound of Example 1, as hereinbefore described.

Example 7

Preparation of 3-Methylamino-1-(5-methyl-2-pyridyl)-2-pyrazoline hydrobromide

Example 7A

3-Amino-1-(5-methyl-2-pyridyl)-2-pyrazoline hydrochloride mono-hydrate

To a solution of sodium (0.915 g) in ethanol (85 ml) stirred at room temperature under nitrogen, was added 5-methyl-2-pyridylhydrazine (9.34 g). The mixture was cooled to −10° and acrylonitrile (5 g) was added slowly with stirring. The mixture was allowed to warm to room temperature and then heated to 80° for 5½ hours. Concentrated hydrochloric acid was added until the mixture was at pH 5, then the mixture was filtered, cooled and treated with ether to precipitate the crude product. Recrystallisation from propan-2-ol afforded 3-amino-1-(5-methyl-2-pyridyl)-2-pyrazoline hydrochloride monohydrate m.p. 277—278°.

The base was obtained by treatment of the hydrochloride with .880 ammonia in the presence of ether.

Example 7B

3-Formamido-1-(5-methyl-2-pyridyl)-2-pyrazoline

3-Amino-1-(5-methyl-2-pyridyl)-2-pyrazoline (2.02 g) was dissolved in 98% formic acid (15 ml) and heated to 60° under a nitrogen atmosphere. To this was added, dropwise, acetic anhydride (3 ml). After heating for one hour at 60° the mixture was evaporated *in vacuo*. The residual yellow oil was dissolved in water and neutralised with .880 ammonia to yield 3-formamido-1-(5-methyl-2-pyridyl)-2-pyrazoline, m.p. 170—171° after recrystallization from toluene.

Example 7C

3-Methylamino-1-(5-methyl-2-pyridyl)-2-pyrazoline hydrobromide

A suspension of 3-formamido-1-(5-methyl-2-pyridyl)-2-pyrazoline (1.49 g) in dry ether (20 ml) was added in portions to a suspension of lithium aluminium hydride (1.05 g) in dry ether (50 ml) and stirred under nitrogen at room temperature.

The mixture was stirred for a further two hours, then decomposed by the dropwise addition of water. The ethereal solution was separated from the inorganic salts, dried over anhydrous potassium carbonate under nitrogen and filtered. Treatment of the ethereal solution with concentrated hydrobromic acid yielded 3-methylamino-1-(5-methyl-2-pyridyl)-2-pyrazoline hydrobromide which was recrystallised from ethanol. m.p. 310°.

Example 8

Preparation of 3-methylamino-1-(6-methyl-2-pyridyl)-2-pyrazoline

Example 8A

3-Amino-1-(6-methyl-2-pyridyl)-2-pyrazoline hydrochloride

6-Methyl-2-pyridylhydrazine (13.06 g) was added to a solution of sodium (1.28 g) in ethanol (120 ml) with stirring at room temperature under nitrogen. The solution was cooled to −5° and acrylonitrile (7 g) was added slowly with stirring. The mixture was allowed to reach ambient temperature and then heated to 80° for five hours.

Concentrated hydrochloric acid (12 ml) was added to the hot reaction mixture, the precipitated sodium chloride removed by filtration, and the filtrate cooled and treated with diethyl ether to precipitate crude 3-amino-1-(6-methyl-2-pyridyl)-2-pyrazoline hydrochloride which was recrystallised from isopropanol, m.p. 261—263°.

Treatment of the hydrochloride with .880 ammonia, in the presence of ether, afforded the base.

Example 8B

3-Formamido-1-(6-methyl-2-pyridyl)-2-pyrazoline

3-Amino-1-(6-methyl-2-pyridyl)-2-pyrazoline (3.3 g) was dissolved in 98% formic acid (24 ml), heated to 60° under a nitrogen atmosphere, and the solution treated dropwise with acetic anhydride (5 ml). The resultant solution was maintained at 60° for three hours before evaporating *in vacuo*. The brown oil obtained was dissolved in methanol and evaporated *in vacuo*; finally the crude product was dissolved in water and the solution neutralised by the addition of .880 ammonia.

The 3-formamido-1-(6-methyl-2-pyridyl)-2-pyrazoline thus obtained was recrystallised from toluene, m.p. 172°—173°.

Example 8C

3-Methylamino-1-(6-methyl-2-pyridyl)-2-pyrazoline hydrobromide dihydrate

3-Formamido-1-(6-methyl-2-pyridyl)-2-pyrazoline (2.21 g), suspended in dry ether (30 ml), was added in portions to a stirred suspension of lithium aluminium hydride (1.23 g) in dry ether (70 ml) under nitrogen at 0°. The reaction mixture was stirred at room temperature for 1½ hours then decomposed by the dropwise addition of water. The ethereal solution was filtered under nitrogen, dried over anhydrous potassium crbonate and again filtered.

Addition of concentrated hydrobromic acid to the ethereal solution yielded 3-methylamino-1-(6-methyl-2-pyridyl)-2-pyrazoline hydrobromide dihydrate as a bright yellow solid, m.p. 245°.

Example 9

Preparation of 3-Ethylamino-1-(6-methyl-2-pyridyl)-2-pyrazoline hydrobromide

Example 9A

3-Acetamido-1-(6-methyl-2-pyridyl)-2-pyrazoline

3-Amino-1-(6-methyl-2-pyridyl)-2-pyrazoline (3.82 g) was dissolved in chloroform (33 ml), stirred under nitrogen, and treated dropwise with acetic anhydride (4.5 ml). The mixture was heated to reflux for 2½ hours.

After cooling, ethanol (4.5 ml) was added and the solution stirred for 30 minutes. The solution was thoroughly extracted with 2N sodium hydroxide solution, and the organic layer dried over anhydrous potassium carbonate before evaporation *in vacuo*. The residue was dissolved in hot ether, treated with decolourising charcoal, filtered, and the filtrate evaporated *in vacuo* to yield 3-acetamido-1-(6-methyl-2-pyridyl)-2-pyrazoline, m.p. 171°.

Example 9B

2-Ethylamino-1-(6-methyl-2-pyridyl)-2-pyrazoline hyrobromide monohydrate

To a suspension of lithium aluminium hydride (1 g) in dry ether (80 ml), stirred at room temperature under nitrogen, was added in portions a suspension of 3-acetamido-1-(6-methyl-2-pyridyl)-2-pyrazoline.

The mixture was heated to reflux for two hours, cooled to 0° and carefully decomposed by the dropwise addition of water. The ethereal solution was decanted and dried over anhydrous potassium carbonate under nitrogen. The drying agent was removed by filtration, and the filtrate treated dropwise with concentrated hydrobromic acid to yield a yellow solid.

This crude product was dissolved in water, reprecipitated with saturated potassium bromide solution and finally recrystallised from a propan-2-ol/ethyl acetate mixture to yield 3-ethylamino-1-(6-methyl-2-pyridyl)-2-pyrazoline hydrobromide (monohydrate), m.p. 153°.

Example 10

Preparation of 3-Methylamino-1-(3-quinolyl)-2-pyrazoline

Example 10A

3-Amino-1-(3-quinolyl)-2-pyrazoline

3-Hydrazinoquinoline (5 g) was added to a solution of sodium (0.7 g) in dry ethanol (25 ml) under an atmosphere of nitrogen at 0° and with stirring. Acrylonitrile (1.8 g) was then added slowly, still at 0° and still

under nitrogen. The resulting mixture was heated to reflux in an oil, when a solid rapidly separated. After 45 minutes the now semi-solid mass was cooled and the separated solid filtered off, washed with methanol and dried *in vacuo*. The resulting 3-amino-1-(3-quinolyl)-2-pyrazoline was a dark yellow solid, m.p. 206—207°.

Example 10B
3-Formamido-1-(3-quinolyl)-2-pyrazoline

3-Amino-1-(3-quinolyl)-2-pyrazoline (1.5 g) (prepared in Example 10A) was added to anhydrous formic acid (9 ml) at 60° under nitrogen and with stirring. Acetic anhydride (2.2 ml) was then added dropwise over a period of 20 minutes, still at 60°. The final mixture was stirred at 60° for a further 30 minutes and treated slowly with water at room temperature. The resulting solid was collected, washed with water and dried *in vacuo* to yield 3-formamido-1-(3-quinolyl)-2-pyrazoline.

Example 10C
2-Methylamino-1-(3-quinolyl)-2-pyrazoline

A suspension of 3-formamido-1-(3-quinolyl)-2-pyrazoline (4.44 g) in dry tetrahydrofuran (4 ml) was added slowly to a stirred suspension of lithium aluminium hydride (2 g) in dry tetrahydrofuran (63 ml) in an atmosphere of nitrogen over a period of 20 minutes at room temperature. The final mixture was kept at room temperature for an additional 45 minutes and then decomposed with ice-cooling with water (8 ml) and 5N-sodium hydroxide (2 ml). The supernatant organic solution was decanted from the inorganic sludge which was washed repeatedly with ether. The combined organic layers were evaporated *in vacuo* and the residual 3-methylamino-1-(3-quinolyl)-2-pyrazoline was purified by repeated crystallisation from aqueous methanol, m.p. 169—172°.

Example 11
Preparation of 1-(4-Chloro-3-trifluoromethylphenyl)-3-methylamino-2-pyrazoline
Example 11A
1-(4-Chloro-3-trifluoromethylphenyl)-3-formamido-2-pyrazoline

3-Amino-1-(4-chloro-3-trifluoromethylphenyl)-2-pyrazoline (1 g) (prepared according to Example 10 of our European patent specification No. 22-578) was added to formic acid at 60° under nitrogen and with stirring. Acetic anhydride (1.2 ml) was then slowly added during 20 minutes (still at 60°) and after a further 15 minutes the mixture was cooled in ice and slowly decomposed with water. The resulting 1-(4-chloro-3-triflouromethylphenyl)-3-formamido-2-pyrazoline was a crystalline solid, m.p. 160—167°, (yield 1 g); it was homogenous by T.l.c.

Example 11B
1-(4-Chloro-3-trifluoromethylphenyl)-3-methylamino-2-pyrazoline hydrochloride

The amide (1 g) (prepared in Example 11A) was added in dry ether suspension to a stirred suspension of lithium aluminium hydride (370 mg) in dry ether (12 ml) in an atmosphere of nitrogen over a period of about 20 minutes. After a further 45 minutes the mixture was decomposed in the usual way at 60° with water (1.5 ml) and 5N sodium hydroxide (0.4 ml). The ethereal layer was decanted, the residual sludge washed with fresh ether, the combined organic extracts were reacted with concentrated hyrochloric acid (0.28 ml) and the mixture evaporated *in vacuo*. The residual 1-(4-chloro-3-trifluoromethylphenyl)-3-methylamino-2-pyrazoline was crystallised by careful precipitation from a solution in methanol with ether, m.p. 176—178°.

Example 12
Preparation of 1-(5-Bromo-6-methyl-2-pyridyl)-3-methylamino-2-pyrazoline
Example 12A
1-(5-Bromo-6-methyl-2-pyridyl)-2-formamido-2-pyrazoline

A mixture of 3-amino-1-(5-bromo-6-methyl-2-pyridyl)-2-pyrazoline (12.5 g) and formic acid (53 ml) was stirred at 60° under nitrogen whilst acetic anhydride (14.3 ml) was added over about 20 minutes. The resulting mixture was maintained at 60° for a further 90 minutes and then evaporated *in vacuo*. The residual 1-(5-bromo-6-methyl-2-pyridyl)-3-formamido-2-pyrazoline was recrystallised from methanol, m.p. 204—207°.

Example 12B
1-(5-Bromo-6-methyl-2-pyridyl)-3-methylamino-2-pyrazoline hydrochloride

A suspension of the amide (2 g) (prepared in Example 12A) in dry tetrahydrofuran (20 ml) was slowly added at room temperature to 1 molar diboran in tetrahydrofuran (42.4 ml) under an atmosphere of nitrogen. After a further 15 minutes at room temperature, aluminium chloride (5.64 g) was added portion-wise over 30 minutes (internal temperature being about 25—35°). The final mixture was stirred at room temperature for 45 minutes and finally heated to reflux for $3\frac{1}{2}$ hours. 6N-hydrochloric acid (7.76 ml) was then added (under nitrogen) and the tetrahydrofuran distilled off pressure. Water (20 ml) was added to the residue followed by excess 10N-sodium hydroxide at 0°. The resulting 1-(5-bromo-6-methyl-2-pyridyl)-3-methylamino-2-pyrazoline was extracted with chloroform:isopropanol (1:1). The solution was dried and evaporated. The residual base was purified by chromatography on an alumina column using ethyl acetate:light petroleum (bp 60—80°) (1:2). The appropriate fraction was evaporated *in vacuo* and the residue (1 g) reacted with oxalic acid (500 mg) in ethanol (5 ml) to give almost pure 1-(5-bromo-6-methyl-2-pyridyl)-3-methylamino-3-pyrazoline hydrogen oxalate. It was recrystallised from a mixture of ethanol and methanol to give yellow crystals, m.p. 108—109°, (yield 750 mg). This oxalate was converted to the corresponding hydrochloride by reaction with excess aqueous hydrochloric acid, followed by evaporation and recrystallisation by precipitation from ethanol and ether to give yellow needles, m.p. 184—185°.

Example 13
Preparation of 3-Dimethylamino-1-(3-trifluoromethylphenyl)-2-pyrazoline
Example 13A
3-N-Methylformamido-1-(3-trifluoromethylphenyl)-2-pyrazoline

3-Methylamino-1-(3-trifluoromethylphenyl)-2-pyrazoline (1 g) (prepared in Example 1) was added to formic acid (6 ml) followed dropwise by acetic anhydride (1.5 ml) at 60° over a period of 10 minutes. Fresh acetic anhydride was then added and the mixture stirred at 60° for a further 10 minutes. Water was then added with ice-cooling and the precipitated 3-N-methylformamido-1-(3-trifluoromethylphenyl)-2-pyrazoline collected and recrystallised from ethanol and water, m.p. 121—122°.

Example 13B
3-Dimethylamino-1-(3-trifluoromethylphenyl)-2-pyrazoline

The amide (4.25 g) (prepared in Example 13A) was added gradually to 1 molar diborane solution in tetrahydrofuran (90.1 ml). Aluminium chloride (12 g) was next added after about 10 minutes stirring. The mixture was kept at room temperature for 15 minutes and then heated to reflux for 3½ hours. Finally, the mixture was cooled and 6N-hydrochloric acid (17 ml) was added and the tetrahydrofuran distilled off at atmospheric pressure. Water was added to the residue and the solution cooled as the mixture was basified with concentrated sodium hydroxide. The crude product was extracted with chloroform:isopropanol (1:1); the extracts were dried over magnesium sulphate and evaporated. The residue was purified by chromatography on an alumina column using ethyl acetate:light petroleum (b.p. 60—80°) (1:4) as the eluant. The pure product solidified and was recrystallised from light petroleum (bp 60—80°), m.p. 85—86°. It was identified as 3-dimethylamino-1-(3-trifluoromethylphenyl)-2-pyrazoline by elementary analysis and mass spectrophotometry.

Example 14
Preparation of 3-ethylamino-5-methyl-1-phenyl-2-pyrazoline

3-Amino-5-methyl-1-phenyl-2-pyrazoline (prepared in Reference Example 14 of our European patent specification No. 22-578) was reacted with acetic anhydride to give 3-acetamido-5-methyl-1-phenyl-2-pyrazoline, m.p. 134—136° as described in Reference Example 32 of the European specification No. 22-578.

This acetamido-compound was then reduced with lithium aluminium hydride according to the method of Example 1, to give 3-ethylamino-5-methyl-1-phenyl-2-pyrazoline, b.p. 125—127°/6,67 Pa.

Example 15
Preparation of 3-butylamino-5-methyl-1-phenyl-2-pyrazoline

According to the method of Example 1, 3-butyramido-5-methyl-1-phenyl-2-pyrazoline (prepared in Example 33 of European patent specification No. 22-578) was reduced to give 3-butylamino-5-methyl-1-phenyl-2-pyrazoline, m.p. 73—75°.

Example 16
3-(1-Phenylethylamino)-1-(3-trifluoromethylphenyl)-2-pyrazoline hydrochloride

To a solution of methyl magnesium iodide, prepared from magnesium turnings (0.97 g) and methyl iodide (5.7 g) in dry ether (20 ml), was added with stirring at room temperature under nitrogen, a suspension of 3-benzylideneamino-1-(3-trifluoromethylphenyl)-2-pyrazoline (3.17 g) in dry ether (110 ml). The resultant mixture was heated to reflux for 2 hours, cooled to room temperature, and poured into a mixture of ice and concentrated hydrochloric acid (10 ml). The ethereal layer was separated, evaporated *in vacuo* and the residual gum was treated with 2N hydrochloric acid to yield 3-(1-phenylethylamino)-1-(3-trifluoromethylphenyl)-2-pyrazoline hydrochloric which was recrystallised from an ethanol/ether mixture, m.p. 192°—193°.

Example 17
Preparation of 3-(2-butenylamino)-1-(2-naphthyl)-2-pyrazoline hydrochloride
Example 17A
3-(2-Butenylideneamino)-1-(2-naphthyl)-2-pyrazoline

3-Amino-1-(2-naphthyl)-2-pyrazoline (1 g) (prepared according to Example 8A of our EP—A—0 056 466 and crotonaldehyde (3.3 g) were heated together at 100° until the mixture became homogeneous. The

resultant solid which separated in cooling was collected and recrystallised from 1-propanol to yield 3-(2-butenylideneamino)-1-(2-naphthyl)-2-pyrazoline.

Example 17B

3-(2-Butenylamino)-1-(2-naphthyl)-2-pyrazoline hydrochloride

3-(2-Butenylideneamino)-1-(2-naphthyl)-2-pyrazoline (620 mg) suspended in methanol (15 ml) was treated with sodium hydroxide (600 mg) added in portions.

The pale green solid product was collected by filtration, dissolved in methanol and neutralised with hydrochloric acid. Evaporation of the solution yielded 3-(2-butenylamino)-1-(2-naphthyl)-2-pyrazoline hydrochloride, which, after recrystallisation from isopropanol/diethyl ether mixture had m.p. 184—185°.

Example 18

Preparation of 3-ethylamino-4-methyl-1-(3-trifluoromethylphenyl)-2-pyrazoline hydrochloride

Example 18A

3-Acetylamino-4-methyl-1-(3-trifluoromethylphenyl)-2-pyrazoline

1.6N Butyl lithium (15.5 ml) was added dropwise at −40° to a solution of 3-amino-4-methyl-1-(3-trifluoromethylphenyl)-2-pyrazoline (6 g) (prepared according to Example 12 of our European patent specification No. 22-578) in dry tetrahydrofuran (50 ml) and stirred under nitrogen. After 10 minutes, ethyl acetate (10 ml) was added and the mixture allowed to warm ambient temperature. The mixture was evaporated down and the residue triturated with ether to yield a solid product which was collected by filtration, m.p. 173—174°.

Example 18B

3-Ethylamino-4-methyl-1-(3-trifluoromethylphenyl)-2-pyrazoline hydrochloride

A suspension of 3-acetylamino-4-methyl-1-(3-trifluoromethylphenyl)-2-pyrazoline (650 mg) in dry ether (10 ml) was added in portions to a suspension of lithium aluminium hydride (330 mg) in dry ether (30 ml) and stirred under dry nitrogen at room temperature. The resultant mixture was stirred for 30 minutes, heated to reflux for 30 minutes, cooled to 0° and a compound by the cautious addition of water. The ether solution was decanted from the precipitated inorganic salts and dried over potassium carbonate under nitrogen. The drying agent was removed by filtration, the filtrate was evaporated *in vacuo* and the gummy base dissolved in methanol and neutralised with hydrochloric acid. Evaporation of the neutral solution yielded a crystalline product which was ground under ethyl acetate and collected to yield 3-ethylamino-4-methyl-1-(3-trifluoromethylphenyl)-2-pyrazoline hydrochloride, m.p. 141—142°.

Example 19

Preparation of 3-benzylamino-1-(3-t-butylphenyl)-2-pyrazoline hydrochloride

A stirred suspension of 3-benzylideneamino-1-(3-t-butylphenyl)-2-pyrazoline (100 mg) (prepared according to Example 13 of our EP—A—0 056 466 in methanol (1 ml) was treated at room temperature with sodium borohydride (50 mg).

After 45 minutes all the suspended solid had dissolved and the red colour had disappeared. The resulting solution was evaporated *in vacuo*, the residue was stirred with water, and the separated oil was extracted with ether. The ether extracts were dried over anhydrous potassium carbonate, filtered and evaporated *in vacuo*. The crude base was dissolved in methanol, neutralised with hydrochloric acid and evaporated *in vacuo* to yield 3-benzylamino-1-(3-t-butylphenyl)-2-pyrazoline hydrochloride which, after crystallisation from isopropanol/diethylether mixture, had m.p. 185—190°.

Example 20

Preparation of 3-benzylamino-1-(3-carboxyphenyl)-2-pyrazoline

Example 20A

3-Benzylideneamino-1-(3-carboxyphenyl)-2-pyrazoline

3-Amino-1-(3-carboxyphenyl)-2-pyrazoline (850 mg) in methanol (10 ml) was treated with benzaldehyde (870 mg) and glacial acetic acid (1 drop). The mixture was heated to reflux for seven hours, then left to cool overnight. The resultant product was filtered off and recrystallised from n-propanol to yield 3-benzylideneamino-1-(3-carboxyphenyl)-2-pyrazoline, m.p. 185°.

Example 20B

3-Benzylamino-1-(3-carboxyphenyl)-2-pyrazoline

A suspension of 3-benzylideneamino-1-(3-carboxyphenyl)-2-pyrazoline (523 mg) in methanol (150 ml) was hydrogenated, at room temperature and pressure, over a platinum oxide catalyst (10 mg). After 50 minutes the uptake of hydrogen was complete.

The catalyst was removed by filtration and the filtrate evaporated *in vacuo* to low volume. Addition of water caused the solid product to crystallise and it was collected by filtration and recrystallised from ethanol/water mixture to yield 3-benzylamino-1-(3-carboxyphenyl)-2-pyrazoline, m.p. 187—190°.

Example 21

Preparation of 3-cyclohexylmethylamino-1-(3-trifluoromethylphenyl)-2-pyrazoline

A solution of 3-amino-1-(3-trifluoromethylphenyl)-2-pyrazoline (9.2 g) (prepared according to Reference Example 1 of our European patent specification No. 22-578 and cyclohexanecarboxylic anhydride (8.8 g) in chloroform (50 ml) was heated to reflux for 5 hours. The resulting mixture was evaporated *in vacuo* to give a gum which was stirred with excess aqueous 2N-sodium hydroxide. The aqueous portion was decanted and the residual gum taken up into ether. This resulting solution was washed twice with water and twice with 4N-hydrochloric acid and then again with water. The residual ethereal solution was dried over magnesium sulphate, filtered and evaporated *in vacuo*. A solution of the resulting gum in a 2:1 mixture of light petroleum (bp 40—60°) and ethyl acetate was percolated down a column of alumina. The first fraction was evaporated to give a crystalline solid which was recrystallised from a very little ethyl acetate and light petroleum to give colorless plates of 3-cyclohexanecarbonamido-1-(3-trifluoromethylphenyl)-2-pyrazoline, m.p. 143—144°.

The amide (2.5 g) was added slowly to a stirred suspension of lithium aluminium hydride (2.0 g) in dried tetrahydrofuran (28 ml) in an atmosphere of nitrogen at 0°. The resulting mixture was gradually heated to reflux for 3 hours after which it was cooled to 0° and very gradually decomposed with water in the usual way. The resulting cyclohexylmethylamino-1-(3-trifluoromethylphenyl)-2-pyrazoline was isolated with ether in the usual way and its hydrochloride was recrystallised from isopropanol and ether, m.p. 163—164°.

Example 22

Preparation of 3-methylamino-1-(2-chlorophenyl)-2-pyrazoline and its hydrochloride

3-Amino-1-(2-chlorophenyl)-2-pyrazoline (prepared according to Reference Example 5 of European patent specification No. 22-578) was reacted with formic acid/acetic anhydride at 60° to give 3-formamido-1-(2-chlorophenyl)-2-pyrazoline m.p. 124—126°.

Reduction of this compound with lithium aluminium hydride in dry ether under nitrogen in the usual way afforded 3-methylamino-1-(2-chlorophenyl)-2-pyrazoline as a solid m.p. 79—83°; its hydrochloride had m.p. 194—196°.

Example 23

Preparation of 3-benzylamino-1-(4-methoxyphenyl)-2-pyrazoline

3-Benzylideneamino-1-(4-methoxyphenyl)-2-pyrazoline (100 mg) prepared according to Example 19 of our EP—A—0 056 466 was suspended in ethanol (3 ml) with stirring whilst sodium borohydride was added. After 1 hour a further 50 mg of sodium borohydride was added. After about 90 minutes the now nearly colourless solution was filtered to remove a trace of insoluble material and the filtrate treated cautiously with water to precipitate pure 3-benzylamino-1-(4-methoxyphenyl)-2-pyrazoline, m.p. 87.5—88°.

Example 24

Preparation of 3-(4-chlorobenzylamino)-1-(4-chlorophenyl)-2-pyrazoline

3-(4-Chlorobenzylideneamino)-1-(4-chlorophenyl)-2-pyrazoline (100 mg) (prepared according to Example 10 of our co-pending application No. A631/A641) was suspended in ethanol (2 ml) and sodium borohydride (100 mg) added with stirring. The deep orange colour disappeared during 30 minutes; addition of water gave 3-(4-chlorobenzylamino)-1-(4-chlorophenyl)-2-pyrazoline as an oil which subsequently crystallised m.p. 105°.

Example 25

Preparation of 1-(4-bromo-3-trifluoromethylphenyl)-3-(4-methoxybenzylamino)-2-pyrazoline and its hydrochloride.

Sodium borohydride (5 mg) was added to a stirred suspension of 1-(4-bromo-3-trifluoromethylphenyl)-3-(4-methoxybenzylideneamino)-2-pyrazoline (100 mg) (prepared according to Example 12 of our EP—A—0 056 466 in isopropanol (20 ml). Two further lots of sodium borohydride (each 5 mg) were added each after 24 and 48 hours respectively. 24 hours after the final addition (total time 72 hours) the now colourless mixture was filtered and the filtrate evaporated *in vacuo*. The residue was extracted with ether and the ethereal solution evaporated to give 1-(4-bromo-3-trifluoromethylphenyl)-3-(4-methoxybenzylamino)-2-pyrazoline which was purified as its hydrochloride m.p. 138°.

Example 26

Preparation of 3-methylamino-1-(3-trifluoromethylphenyl)-2-pyrazoline *p*-toluenesulphonate

3-Methylamino-1-(3-trifluoromethylphenyl)-2-pyrazoline (243 mg) (prepared according to Example 1 above) was reacted with *p*-toluenesulphonic acid (210 mg) is isopropanol (5 ml) in an atmosphere of nitrogen. The resulting solution was evaporated *in vacuo* and the residue ground up with a mixture of ethyl acetate and light petroleum (b.p. 60—80°) to give 3-methylamino-1-(3-trifluoromethylphenyl)-2-pyrazoline *p*-toluenesulphonate, m.p. 155—156°.

Example 27

3-Amino-1-(4-chlorophenyl)-2-pyrazoline (1.8 g) (prepared according to Reference Example 5 of European patent specification No. 22-587) was dissolved in anhydrous formic acid (10 ml) at room temperature in an atmosphere of nitrogen. Acetic anhydride (2.66 ml) was added dropwise over a period of 5 minutes and the resulting mixture, still under nitrogen, was heated at 60° for 15 minutes. Water was then added at about 20° until a crystalline solid separated. The resulting 3-formamido-1-(4-chlorophenyl)-2-pyrazoline was collected and recrystallised from methanol, m.p. 154—155° (yield 1.425 g).

Analysis: $C_{10}H_{10}ClN_3O$

| | | | |
|---|---|---|---|
| Required: | C, 53.70; | H, 4.51; | N, 18.79 |
| Found: | C, 53.89; | H, 4.51; | N, 18.48 |

A solution of the formamido compound (1.425 g) in dry tetrahydrofuran (15 ml) was added to a stirred suspension of lithium aluminium hydride (1.74 g) in dry tetrahydrofuran (1.74 g) in an atmosphere of nitrogen at 0°. The resulting mixture was stirred at room temperature for 2 hours and was then cautiously decomposed with water in the usual way, still under nitrogen. The organic layer was separated and the residual aqueous sludge extracted twice with ether. The combined organic layers were dried over potassium carbonate, and evaporated. The residue was reacted with a slight excess of concentrated hydrochloric acid in methanol, when evaporation gave 3-methylamino-1-(4-chlorophenyl)-2-pyrazoline hydrochloride. It was recrystallised from isopropanol, m.p. 168—169°, (yield 1.187 g).

Analysis: $C_{10}H_{12}ClN_3HCl$

| | | | |
|---|---|---|---|
| Required: | C, 48.8; | H, 5.32; | N, 17.07 |
| Found: | C, 48.15; | H, 5.41; | N, 17.10 |

Example 28

3-Amino-1-(2-pyridyl)-2-pyrazoline (1.5 g) (prepared according to Example 25 of European patent specification No. 22-578) was added to formic acid (22 ml) at 60° in an atmosphere of nitrogen. The mixture was stirred whilst acetic anhydride (2.3 ml) was added dropwise. The mixture was heated at 100° for 1 hour before being evaporated *in vacuo* to give 3-formamido-1-(2-pyridyl)-2-pyrazoline which was recrystallised from isopropanol, m.p. 182—183°, (yield 1.17 g).

A solution of the formamido compound (1.15 g) in dry tetrahydrofuran (100 ml) was added dropwise to a stirred suspension of lithium aluminium hydride (1.65 g) in dry tetrahydrofuran (16.5 ml) in an atmosphere of nitrogen. After stirring for 1 hour at room temperature, the final mixture was heated to reflux for 2 hours before being decomposed with water in the usual way. The organic layer was then separated, the residue washed twice with fresh ether and the combined organic layers dried over potassium carbonate before filtration and evaporation. The resulting crude base was taken up into methanol and a slight excess of 2N-hydrochloric acid (3.1 ml) was added. Evaporation gave a rather sticky product which solidified on trituration with isopropanol. The resulting 3-methylamino-1-(2-pyridyl)-2-pyrazoline hydrochloride was a yellow solid, m.p. 263°, (yield 200 mg).

Analysis: $C_9H_{13}ClN_4$

| | | | | |
|---|---|---|---|---|
| Required: | C, 50.82; | H, 6.16; | N, 26.34; | Cl, 16.67 |
| Found: | C, 50.36; | H, 6.29; | N, 25.87; | Cl, 16.25 |

Example 29

Isobutyric anhydride (5 ml) was added to a solution of 3-amino-1-(3-trifluoromethylphenyl)-2-pyrazoline (5 g) (prepared according to Reference Example 1 of European patent specification No. 22-578) in chloroform (30 ml) and the mixture heated to reflux for 1 hour. The excess anhydride was decomposed by the addition of ethanol (5 ml) and refluxed for a further 30 minutes. The cooled mixture was extracted with 2N-sodium hydroxide, dried over potassium carbonate and evaporated to give a dark brown gum which crystallised over a period of days. Trituration with ether gave a dark coloured solid m.p. 164° (2.265 g) which was recrystallised from light petroleum (b.p. 80—100°) to give 3-isobutyramido-1-(3-trifluoromethylphenyl)-2-pyrazoline (1.77 g) m.p. 167—168°.

A solution of the isobutyramido compound (1.72 g) in dry tetrahydrofuran (15 ml) was added dropwise to a suspension of lithium aluminium hydride (1.5 g) in dry tetrahydrofuran (15 ml) at 0° and in an atmosphere of nitrogen. After stirring at room temperature for 2 hours, the mixture was heated to reflux for 2 hours. It was finely cooled and decomposed with water in the usual way. The organic layer was separated, the residual sludge washed twice with ether and all the combined extracts dried over potassium carbonate, then filtered and evaporated. The residue was reacted with a slight excess of 2N-hydrochloric acid to give 3-(3-methylpropylamino)-1-(3-trifluoromethylphenyl)-2-pyrazoline hydrochloride, m.p. 143—144° (1.0 g).

Analysis: $C_{14}H_{18}F_3N_3$

| | | | |
|---|---|---|---|
| Required: | C, 50.26; | H, 5.95; | N, 13.06 |
| Found: | C, 52.53; | H, 6.04; | N, 13.00 |

Example 30

Preparation of the starting material for Example 9A

A mixture of 2-bromo-6-methyl pyridine (36.2 mg), butanol (36 ml) and hydrazine hydrate (110 ml) was heated in an oil bath at 130—135° for 5 hours. Evaporation *in vacuo* then gave a residue which was treated with excess 10-N sodium hydroxide (50 ml) to give an oil. This oil was isolated in the usual way to give 6-methyl-2-pyridyl hydrazine, which was purified by distillation, b.p. 90—100° (6,67 Pa) and subsequently crystallised, m.p. 51.2°.

6-Methyl-2-pyridyl hydrazine (13.06 g) was added to a stirred solution of sodium (1.28 g) in ethanol (120 ml) in an atmosphere of nitrogen. This mixture was allowed to warm to room temperature, and after a further 30 minutes, it was heated to 80° for 5 hours. Concentrated hydrochloric acid was then added whilst still hot, and the precipitated sodium chloride removed by filtration. Addition of ether to the cooled filtrate produced 3-amino-1-(6-methyl-2-pyridyl)-2-pyrazoline hydrochloride, m.p. (after drying at 100 *in vacuo*) was 216—263° (yield 8.2 g).

Example 31

3-Amino-1-(2-naphthyl)-2-pyrazoline

2-Hydrazinonaphthalene (5 g) was added to a solution of sodium (0.7 g) in dried spiritus vitalis methanol (S.V.M.) (20 ml) in a nitrogen atmosphere at 0—5°. Acrylonitrile (1.8 g) was then added slowly and the resulting mixture allowed at attain room temperature over about 1 hour.

The mixture was then heated to reflux; after about 30 minutes it deposited a crystalline solid and after about 45 minutes a semi-solid mass had formed. After a total heating time of 1 hour, the mixture was allowed to cool and the solid filtered off with care.

The filtrate was deep purple but the residue was a clear yellow solid which was ground up with water, filtered and re-ground with S.V.M. After further filtering and grinding with S.V.M., the product was finally filtered, washed with S.V.M. and dried *in vacuo* to yield 4.4 g of 3-amino-1-(2-naphthyl)-2-pyrazoline, m.p. 190—191°.

Example 32

Preparation of 3-(4-chlorobenzylideneamino)-1-(4-chlorophenyl)-2-pyrazoline

3-Amino-1-(4-chlorophenyl)-2-pyrazoline (prepared in Reference Example 6 of our European Patent Specification No. 22-578) (1.95 g) in S.V.M. (5 ml) was mixed with excess 4-chlorobenzaldehyde (1.50 g) and the mixture was heated to reflux after the addition of 1 drop of glacial acetic acid. A virtually clear solution formed which rapidly crystallised to form a bright orange product which was collected, washed with S.V.M. and dried *in vacuo* to produce 2.95 g 3-(4-chlorobenzylideneamino)-1-(4-chlorophenyl)-2-pyrazoline, m.p. 193—195° (decomp).

Example 33

Preparation of 1-(4-bromo-3-trifluoromethylphenyl)-3-(4-methoxybenzylideneamino)-2-pyrazoline

3-Amino-1-(4-bromo-3-trifluoromethylphenyl)-2-pyrazoline (prepared in Example 39 of our European Patent Specification No. 22-578) (240 mg) in S.V.M. (5 ml) together with 4-methoxybenzaldehyde (160 mg) and 1 drop of glacial acetic acid were heated to reflux for 1 hour. A yellow product separated which was collected, washed with methanol and dried *in vacuo* to yield 300 mg 1-(4-bromo-3-trifluoromethylphenyl)-3-(4-methoxybenzylideneamino)-2-pyrazoline, m.p. 175—176° (decomp).

Example 34

Preparation of 3-benzylideneamino-1-(3-t-butylphenyl)-2-pyrazoline

The title compound was prepared in a manner analogous to that described in Example 20A.

Example 35

3-Benzylideneamino-1-(4-methoxyphenyl)-2-pyrazoline, m.p. 199—200°.

The title compound was prepared in a manner analogous to that described in Example 20A.

Example A

Tablet

| | In 1 tablet |
|---|---|
| Active ingredient | 5.0 mg |
| Lactose | 82.0 mg |
| Starch | 10.0 mg |
| Povidone(R) | 2.0 mg |
| Magnesium stearate | 1.0 mg |

Mix together the active ingredient, lactose and starch. Granulate the powders using a solution of povidone in purified water. Dry the granules, add the magnesium stearate and compress to produce tablets, 100 mg per tablet.

Example B
Ointment

| | |
|---|---|
| Active ingredient | 1.0 g |
| White soft paraffin | to 100.0 g |

Disperse the active ingredient in a small volume of the vehicle. Gradually incorporate this into the bulk to produce a smooth, homogeneous product. Fill into collapsible metal tubes.

Example C
Cream for topical use

| | |
|---|---|
| Active ingredient | 1.0 g |
| Polawax GP 200(R) | 20.0 g |
| Lanolin anhydrous | 2.0 g |
| White beeswax | 2.5 g |
| Methyl hydroxybenzoate | 0.1 g |
| Distilled water | to 100.0 g |

Heat the Polawax, beeswax and lanolin together at 60°C. Add a solution of methyl hydroxybenzoate. Homogenise using high speed stirring. Allow the temperature to fall to 50°. Add and disperse the active ingredient. Allow to cool with slow speed stirring.

Example D
Lotion for topical use

| | |
|---|---|
| Active ingredient | 1.0 g |
| Sorbitan monolaurate | 0.6 g |
| Polysorbate 20(R) | 0.6 g |
| Cetostearyl alcohol | 1.2 g |
| Glycerin | 6.0 g |
| Methyl hydroxybenzoate | 0.2 g |
| Purified water b.p. | to 100.00 ml |

The methyl hyroxybenzoate and glycerin were dissolved in 70 ml of the water at 75°C. The sorbitan monolaurate, Polysorbate 20 and cetostearyl alcohol were melted together at 75°C and added to the aqueous solution. The resulting emulsion was homogenised, allowed to cool with continuous stirring and the active ingredient added as a suspension in the remaining water. The whole was stirred until homogeneous.

Example E
Eye drops

| | |
|---|---|
| Active ingredient | 0.5 g |
| Methyl hydroxybenzoate | 0.01 g |
| Propyl hydroxybenzoate | 0.04 g |
| Purified water b.p. | to 100.00 ml |

The methyl and propyl hydroxybenzoates were dissolved in 70 ml purified water at 75° and the resulting solution then allowed to cool. The active ingredient was added next and the solution made up to

100 ml with purified water. The solution was sterilised by filtration through a membrane filter 0.22 μm pore size and packed aseptically into suitable sterile containers.

Example F
Injection solution

| | |
|---|---|
| Active ingredient | 10.0 mg |
| Water for injections b.p. | to 1.0 ml |

The active ingredient was dissolved in half of the Water for Injection and then made up to volume and sterilised by filtration. The resulting solution was distributed into ampoules under aseptic conditions.

Example I
Inhibition of lipoxygenase and cyclo-oxygenase

In an enzyme assay according to the method of G. Blackwell and R. J. Flower (Br. J. Pharmac., *63*: 360P (1978)), compounds of the invention were found to have an $IC_{50}$ (μM) for inhibition of each of lipoxygenase and cyclooxygenase as indicated in Table I.

TABLE I

| Compound | IC$_{50}$ (μM) | |
|---|---|---|
| | Cyclo-oxygenase | Lipoxygenase |
| of Example 1 | 1.7 | 0.4 |
| of Example 2 | 0.8 | 0.7 |
| of Example 3 | 0.1 | 0.1 |
| of Example 4 | 0.3 | 0.3 |
| of Example 5 | 0.3 | 0.3 |
| of Example 7 | >10 | 3 |
| of Example 8 | 40 | 5 |
| of Example 9 | 5 | >10 |
| of Example 10 | >10 | >10 |
| of Example 11 | 20.2 | ~0.1 |
| of Example 12 | 0.3 | 0.6 |
| of Example 13 | 5 | 10 |
| of Example 14 | ~1 | <1 |
| of Example 15 | >10 | >10 |
| of Example 16 | 2 | 2 |
| of Example 17 | 0.1 | 0.025 |
| of Example 18 | ~1 | <1 |
| of Example 20 | >100 | 40 |
| of Example 21 | ~1 | <1 |
| of Example 22 | >10 | 9 |
| of Example 23 | ≪10 | ≪10 |
| of Example 24 | 0.3 | <0.1 |
| of Example 25 | <1 | <1 |

Example II
In vivo activity

The compound of Example 1 was tested as follows (all results are given as $ED_{50}$ mg/kg upon oral administration):

TABLE II

| Test | Compound of Example 1 |
|---|---|
| Prostaglandin production (rat) | 20.0 |
| Leukocyte migration (rat) | 20.0 |
| Carageenin Oedema (rat) | 26.0 |
| PBQ writhing (mouse) | 20.0 |

Example III
Local ophthalmic activity

The anti-inflammatory activity of the compound of Example 1 in the rat eye was compared with that of Dexamethasone, Flurbiprofen, Indomethacin and Oxyphenbutazone. The inflammatory response in this test developed following injection of gram-negative bacterial endotoxin into the rat foot pad (Rosenbaum *et al*, Nature, *286*, 611—613 (1980)). Rats were treated by topical application three times per day and vasodilation was assessed at 24 hour or 48 hour using a slit-lamp. The rats were then killed and the aqueous humour was aspirated for determination of protein concentrations and total leukocyte numbers. Results are shown in Table III as $ED_{50}$ (µg/eye).

TABLE III

| Compound | Inhibition of vasodilatation | Inhibition leukocyte administration |
|---|---|---|
| of Example 1 | 5.5 | 2.6** |
| Dexamethasone | 0.42 | 0.17 |
| Flurbiprofen | >10.0 | 2.1* |
| Indomethacin | >10.0 | >10.0 |
| Oxyphenbutazone | >25.0 | 12.0 |

*at high doses of flurbiprofen, this effect was reversed.
**at high doses (e.g. 10 µg/eye) of the compound of Example 1, this effect was maintained.

Example IV
Antispasmogenic action

In the presence of indomethacin (2.8 µmol), arachidonic acid (3—100 µmol) produces a concentration-dependent contraction of guinea-pig tracheal rings *in vitro*. The concentration of the compound of Example 1 which produces 50% inhibition of the contractile response to arachidonic acid (100 µmol) under the above conditions is ≃8 µmol.

Example V
Toxicity

A. It was found that the $LD_{50}$ of the compound of Example 1 in the rat is 500 mg/kg and in the mouse is 500 mg/kg. The $LD_{50}$ dose of the compound of Example I is therefore at least 25 times greater than its therapeutic dose in both rats and mice.

B. The compound of Example I dose not inhibit the generation of prostaglandis in the stomach and at doses up to 100 mg/kg is not ulcerogenic in the rat.

C. Repeated oral administration of the compound of Example I (125 mg/kg day) to rabbits did not cause any significant haematological changes.

**Claims**

1. A pharmaceutical formulation comprising an active ingredient in association with a pharmaceutically acceptable carrier therefor, characterised in that the active ingredient is a compound of formula (I):

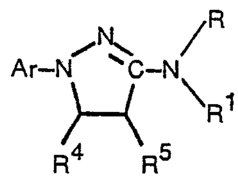

(I)

wherein

Ar is a group selected from phenyl, naphthyl, quinolyl and pyridyl, which group may be optionally substituted by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms;

R is selected from hydrogen, $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl;

$R^1$ is selected from $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl; or an acid addition salt thereof.

2. A formulation according to claim 1, characterised in that in formula (I), R is selected from hydrogen, $C_{1-6}$ alkyl, (optionally substituted by a substituent selected from phenyl and cycloalkyl of from 3 to 6 carbon atoms), $C_{2-6}$ alkenyl and $C_{2-6}$ alkynyl; and $R^1$ is selected from $C_{1-6}$ alkyl (optionally substituted by a substituent selected from phenyl and cycloalkyl of from 3 to 6 carbon atoms), $C_{2-6}$ alkenyl and $C_{2-6}$ alkynyl.

3. A formulation according to Claim 1, characterised in that the active ingredient is selected from
3-methylamino-1-(3-trifluoromethylphenyl)-2-pyrazoline;
3-ethylamino-1-(3-trifluoromethylphenyl)-2-pyrazoline;
3-propylamino-1-(3-trifluoromethylphenyl)-2-pyrazoline;
3-butylamino-1-(3-trifluoromethylphenyl)-2-pyrazoline;
3-benzylamino-1-(3-trifluoromethylphenyl)-2-pyrazoline hydrochloride;
3-(N,N-dimethylamino)-1-(4-trifluoromethylphenyl)-2-pyrazoline;
3-(N-methyl-N-ethylamino)-1-(4-chlorophenyl)-2-pyrazoline;
3-(methylamino)-1-(4-fluorophenyl)-5-methyl-2-pyrazoline;
3-(benzylamino)-1-(4-bromophenyl)-4-methyl-2-pyrazoline;
3-(ethylamino)-1-(3-trifluoromethyl-4-fluorophenyl)-2-pyrazoline;
3-(tert-butylamino)-1-(3-trifluoromethyl-4-bromophenyl)-2-pyrazoline;
3-(isopropylamino)-1-(5-chloro-2-pyridyl)-2-pyrazoline;
3-(methylamino)-1-(5-bromo-2-pyridyl)-2-pyrazoline;
3-(benzylamino)-1-(5-iodo-2-pyridyl)-2-pyrazoline;
3-(allylamino)-1-(3-trifluoromethylphenyl)-2-pyrazoline;
3-(cyclohexylmethylamino)-1-(3-trifluoromethylphenyl)-2-pyrazoline;
3-methylamino-1-(5-methyl-2-pyridyl)-2-pyrazoline;
3-methylamino-1-(6-methyl-2-pyridyl)-2-pyrazoline;
3-ethylamino-1-(6-methyl-2-pyridyl)-2-pyrazoline;
3-methylamino-1-(3-quinolyl)-2-pyrazoline;
3-methylamino-1-(4-chloro-3-trifluoromethylphenyl)-2-pyrazoline;
3-methylamino-1-(5-bromo-6-methyl-2-pyridyl)-2-pyrazoline;
3-(N,N-dimethylamino)-1-(3-trifluoromethylphenyl)-2-pyrazoline;
3-ethylamino-5-methyl-1-phenyl-2-pyrazoline;
3-butylamino-5-methyl-1-phenyl-2-pyrazoline;
3-(1-phenylethylamino)-1-(3-trifluoromethylphenyl)-2-pyrazoline;
3-(2-butenylamino)-1-(2-naphthyl)-2-pyrazoline;
3-ethylamino-4-methyl-1-(3-trifluoromethylphenyl)-2-pyrazoline;

3-benzylamino-1-(3-t-butylphenyl)-2-pyrazoline;
3-benzylamino-1-(3-carboxyphenyl)-2-pyrazoline;
3-methylamino-1-(2-chlorophenyl)-2-pyrazoline;
3-benzylamino-1-(4-methoxyphenyl)-2-pyrazoline;
3-(4-chlorobenzylamino-1-(4-chlorophenyl)-2-pyrazoline; and
3-(4-methoxybenzylamino)-1-(4-bromo-3-trifluoromethylphenyl)-2-pyrazoline.

4. A pharmaceutical formulation comprising an active ingredient in association with a pharmaceutically acceptable carrier therefor, characterised in that the active ingredient is 3-(methylamino)-1-(3-trifluoromethylphenyl)-2-pyrazoline or a pharmaceutically acceptable acid addition salt thereof.

5. A formulation according to any of Claims 1 to 4, characterised by being in a form suitable for ophthalmic administration.

6. A formulation according to any of Claims 1 to 5, characterised by being in the form of aqueous eye drops.

7. A formulation according to any of Claims 1 to 6, characterised in that the active ingredient is further in association with another therapeutic ingredient selected from anti-biotic, anti-fungal and anti-viral agents.

8. A compound of formula (I), as defined in any of Claims 1 to 7, for use in the prophylaxis or treatment of a medical condition associated with inflammation.

9. A compound of formula (I), as defined in any of Claims 1 to 7, for use in the prophylaxis or treatment of a medical condition selected from arthritic conditions, inflammatory skin conditions, inflammatory eye conditions, tissue necrosis, tissue rejection following transplant surgery, pain, pyresis and asthma.

10. A compound of formula (I), as defined in any of Claims 1 to 7, for use in medicine in the ihibition of the lipoxygenase or cyclo-oxygenase pathways of arachidonic acid metabolism.

11. 3-Methylamino-1-(3-trifluoromethylphenyl)-2-pyrazoline for use in the prophylaxis or treatment of inflammatory eye conditions.

12. A compound of the formula (I):

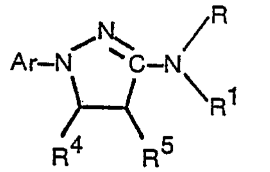

(I)

characterised in that

Ar is a group selected from phenyl, naphthyl, quinolyl and pyridyl, which group may be optionally substituted by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms;

R is selected from hydrogen, $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl;

$R^1$ is selected from $C_{1-6}$ alkyl (optionally substituted by a substituent selected from phenyl which may itself be substituted in any position of the ring by one or more substituents selected from halo, $C_{1-6}$ alkyl (which may itself be substituted by one or more halogen atoms), carboxy, $C_{1-6}$ alkoxy, amino (which may itself be optionally substituted by 1 or 2 $C_{1-6}$ alkyl groups), hydroxy, and $C_{1-6}$ alkylsulphonyl in which the alkyl moiety may be optionally substituted by one or more halogen atoms, and cycloalkyl of from 3 to 6 carbon atoms), $C_{2-6}$ alkenyl and $C_{2-6}$ alkynyl; and

$R^4$ and $R^5$ are the same or different and are each selected from hydrogen and $C_{1-6}$ alkyl;

or an acid addition salt thereof PROVIDED THAT when Ar is unsubstituted-phenyl AND

EITHER (a) when R is methyl, THEN $R^1$ is other than methyl

OR (b) when R is ethyl, THEN $R^1$ is other than both hydrogen and ethyl.

13. A compound according to Claim 12, characterised by being of formula (ID):

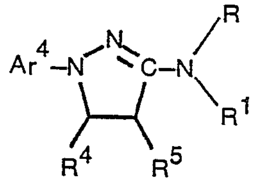

(ID)

wherein

$Ar^4$ is a monocyclic aromatic radical selected from pyridyl which is optionally substituted in any position of the ring by one or more substituent(s), and phenyl substituted in any position of the ring by one or more substituent(s);

R is selected from hydrogen and $C_{1-6}$ alkyl (optionally substituted by phenyl);

$R^1$ is $C_{1-6}$ alkyl (optionally substituted by phenyl); and

$R^4$ and $R^5$ are the same or different and are each selected from hydrogen and $C_{1-6}$ alkyl;

and acid addition salts thereof.

14. 3-(Methylamino)-1-(3-trifluoromethylphenyl)-2-pyrazoline and acid addition salts thereof.

15. A method for preparing a compound of formula (I), as defined in Claim 12, characterised by

(a) cyclisation and elimination of water from a compound of formula (II):

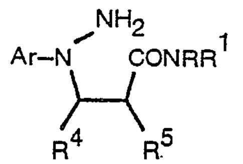

(II)

wherein R, $R^1$, $R^4$, $R^5$ and Ar are as defined in formula (I); or by

(b) reaction of a compound of formula (VII) with $R^8Y$:

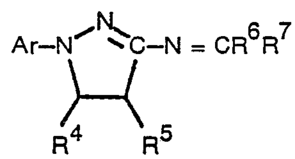

(VII)

wherein Ar, $R^4$ and $R^5$ are as defined in formula (I), Y is an alkali metal or an alkaline earth metal halide, and $—CR^6R^7R^8$ is $R^1$ as defined in formula (I); or by

(c) reduction of a compound of formula (VII), wherein Ar, $R^4$ and $R^5$ are as defined in formula (I), and $—CHR^6R^7$ is $R^1$ as defined in formula (I); or by

(d) reduction of a compound of formula (X):

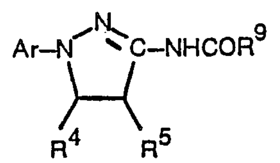

(X)

wherein Ar, $R^4$ and $R^5$ are as defined in formula (I), and either $CH_2R^9$ is $R^1$ as defined in formula (I) or, when $R^1$ is methyl, $R^9$ may be alkoxy;

and optionally converting any compound of formula (I) so prepared to any other compound of formula (I).

**Patentansprüche**

1. Pharmazeutische Zubereitung, die einen aktiven Bestandteil in Assoziation mit einem pharmazeutisch unbedenklichen Träger dafür enthält, dadurch gekennzeichnet, daß der aktive Bestandteil eine Verbindung der Formel I

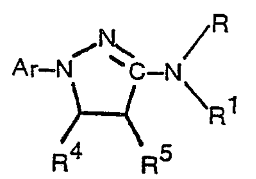

(I)

ist, worin

Ar eine Gruppe ist, die ausgewählt ist unter Phenyl, Naphthyl, Chinolyl und Pyridyl, wobei die Gruppe gegebenenfalls substituiert sein kann durch einen oder mehrere Substituenten, die ausgewählt sind unter Halogen, $C_{1-6}$-Alkyl (welches selbst durch ein oder mehrere Halogenatome (substituiert sein kann), Carboxy, $C_{1-6}$-Alkoxy, Amino (welches selbst gegebenenfalls durch ein oder zwei $C_{1-6}$-Alkylreste substituiert sein kann), Hydroxy, und $C_{1-6}$-Alkylsulfonyl, worin die Alkyleinheit gegebenenfalls durch ein oder mehrere Halogenatome substituiert sein kanne,

R ausgewählt ist unter Wasserstoff, $C_{1-6}$-Alkyl (gegebenenfalls substituiert durch einen Substituenten, der ausgewählt ist unter Phenyl, welches selbst in einer beliebigen Position des Ringes substituiert sein

kann durch ein oder mehrere Substituenten, die ausgewählt sind unter Halogen, $C_{1-6}$-Alkyl (welches selbst substituiert sein kann durch ein oder mehrere Halogenatome), Carboxy, $C_{1-6}$-Alkoxy, Amino (welches selbst gegebenenfalls durch ein oder zwei $C_{1-6}$-Alkylreste substituiert sein kann), Hydroxy, und $C_{1-6}$-Alkylsulfonyl, worin die Alkyleinheit gegebenenfalls substituiert sein kann durch ein oder mehrere Halogenatome, und Cycloalkyl mit 3 bis 6 Kohlenstoffatomen), $C_{2-6}$-Alkenyl und $C_{2-6}$-Alkinyl,

$R^1$ ausgewählt ist unter $C_{1-6}$-Alkyl (gegebenenfalls substituiert durch einen Substituenten, der ausgewählt ist unter Phenyl, welches selbst in einer beliebigen Position des Ringes substituiert sein kann durch einen oder mehrere Substituenten, die ausgewählt sind unter Halogen, $C_{1-6}$-Alkyl (welches selbst substituiert sein kann durch ein oder mehrere Halogenatome), Carboxy, $C_{1-6}$-Alkoxy, Amino (welches selbst gegebenenfalls substituiert sein kann durch ein oder zwei $C_{1-6}$-Alkylreste), Hydroxy, und $C_{1-6}$-Alkylsulfonyl, worin die Alkyleinheit gegebenenfalls substituiert sein kann durch ein oder mehrere Halogenatome, und Cycloalkyl mit 3 bis 6 Kohlenstoffatomen), $C_{2-6}$-Alkenyl und $C_{2-6}$-Alkinyl, und

$R^4$ und $R^5$ gleich oder unterschiedlich sind und jeweils ausgewählt sind unter Wasserstoff und $C_{1-6}$-Alkyl,

oder ein Säureadditionssalz davon.

2. Zubereitung nach Anspruch 1, dadurch gekennzeichnet, daß in der Formel I R ausgewählt ist unter Wasserstoff, $C_{1-6}$-Alkyl (gegebenenfalls substituiert durch einen substituenten, der ausgewählt ist unter Phenyl und Cycloalkyl mit 3 bis 6 Kohlenstoffatomen), $C_{2-6}$-Alkenyl und $C_{2-6}$-Alkinyl, und $R^1$ ausgewählt ist unter $C_{1-6}$-Alkyl (gegebenenfalls substituiert durch einen Substituenten, der ausgewählt ist unter Phenyl und Cycloalkyl mit 3 bis 6 Kohlenstoffatomen), $C_{2-6}$-Alkenyl und $C_{2-6}$-Alkinyl.

3. Zubereitung nach Anspruch 1, dadurch gekennzeichnet, daß der aktive Bestandteil ausgewählt ist unter

3-Methylamino-1-(3-trifluormethylphenyl)-2-pyrazolin,
3-Ethylamino-1-(3-trifluormethylphenyl)-2-pyrazolin,
3-Propylamino-1-(3-trifluormethylphenyl)-2-pyrazolin,
3-Butylamino-1-(3-trifluormethylphenyl)-2-pyrazolin,
3-Benzylamino-1-(3-trifluormethylphenyl)-2-pyrazolinhydrochlorid,
3-(N,N-Dimethylamino)-1-(4-trifluormethylphenyl)-2-pyrazolin,
3-(N-Methyl-N-ethylamino)-1-(4-chlorphenyl)-2-pyrazolin,
3-(Methylamino)-1-(4-fluorphenyl)-5-methyl-2-pyrazolin,
3-(Benzylamino)-1-(4-bromphenyl)-4-methyl-2-pyrazolin,
3-(Ethylamino)-1-(3-trifluormethyl-4-fluorphenyl)-2-pyrazolin,
3-(tert.-Butylamino)-1-(3-trifluormethyl-4-bromphenyl)-2-pyrazolin,
3-(Isopropylamino)-1-(5-chlor-2-pyridyl)-2-pyrazolin,
3-(Methylamino)-1-(5-brom-2-pyridyl)-2-pyrazolin,
3-(Benzylamino)-1-(5-Jod-2-pyridyl)-2-pyrazolin,
3-(Allylamino)-1-(3-trifluormethylphenyl)-2-pyrazolin,
3-(Cyclohexylmethylamino)-1-(3-trifluormethylphenyl)-2-pyrazolin,
3-Methylamino-1-(5-methyl-2-pyridyl)-2-pyrazolin,
3-Methylamino-1-(6-methyl-2-pyridyl)-2-pyrazolin,
3-Ethylamino-1-(6-methyl-2-pyridyl)-2-pyrazolin,
3-Methylamino-1-(3-chinolyl)-2-pyrazolin,
3-Methylamino-1-(4-chlor-3-trifluormethylphenyl)-2-pyrazolin,
3-Methylamino-1-(5-brom-6-methyl-2-pyridyl)-2-pyrazolin,
3-(N,N-Dimethylamino)-1-(3-trifluormethylphenyl)-2-pyrazolin,
3-Ethylamino-5-methyl-1-phenyl-2-pyrazolin,
3-Butylamino-5-methyl-1-phenyl-2-pyrazolin,
3-(1-Phenylethylamino)-1-(3-trifluormethylphenyl)-2-pyrazolin,
3-(2-Butenylamino)-1-(2-naphthyl)-2-pyrazolin,
3-Ethylamino-4-methyl-1-(3-trifluormethylphenyl)-2-pyrazolin,
3-Benzylamino-1-(3-tert.-butylphenyl)-2-pyrazolin,
3-Benzylamino-1-(3-carboxyphenyl)-2-pyrazolin,
3-Methylamino-1-(2-chlorphenyl)-2-pyrazolin,
3-Benzylamino-1-(4-methoxyphenyl)-2-pyrazolin,
3-(4-Chlorbenzylamino)-1-(4-chlorphenyl)-2-pyrazolin und
3-(4-Methoxybenzylamino)-1-(4-brom-3-trifluormethylphenyl)-2-pyrazolin.

4. Pharmazeutische Zubereitung, die einen aktiven Bestandteil in Assoziation mit einem pharmazeutisch unbedenklichen Träger dafür enthält, dadurch gekennzeichnet, daß der aktive Bestandteil 3-(Methylamino)-1-(3-trifluormethylphenyl)-2-pyrazolin oder ein pharmazeutisch unbedenkliches Säureadditonssalz davon ist.

5. Zubereitung nach einem der Ansprüche 1 bis 4, dadurch gekennzeichnet, daß sie in einer Form vorliegt, die für die ophthalmische Verabfolgung geeignet ist.

6. Formulierung nach einem der Ansprüche 1 bis 5, dadurch gekennzeichnet, daß sie in der Form von wässrigen Augentropfen vorliegt.

7. Zubereitung nach einem der Ansprüche 1 bis 6, dadurch gekennzeichnet, daß der aktive Bestandteil

ferner in Assoziation mit einem anderen therapeutischen Bestandteil vorliegt, der ausgewählt ist unter antibiotischen, antifungalen und antiviralen Mitteln.

8. Verbindung nach Formel I gemäß der Definition in einem der Ansprüche 1 bis 7 zur Verwendung in der Prophylaxe oder zur Behandlung eines Krankheitszustandes, der mit einer Entzündung verbunden ist.

9. Verbindung nach Formel I gemäß der Definition in einem der Ansprüche 1 bis 7 zur Verwendung in der Prophylaxe oder zur Behandlung eines Erkrankungszustandes, der ausgewählt ist unter arthritischen Erkrankungen, Entzündungen der Haut, Entzündungen des Auges, Gewebenecrose, Gewebe-Abstoßung im Anschluß an eine Transplantationsoperation, Schmerzen, Pyrese und Asthma.

10. Verbindung nach Formel I gemäß der Definition in einem der Ansprüche 1 bis 7 zur Verwendung in der Medizin bei der Inhibierung des Lipoxygenase- oder Cyclooxygenase-Weges des Arachidonsäure-Metabolismus.

11. 3-Methylamino-1-(3-trifluormethylphenyl)-2-pyrazolin zur Verwendung in der Prophylaxe oder zur Behandlung von Augen-Entzündungen.

12. Verbindung der Formel I

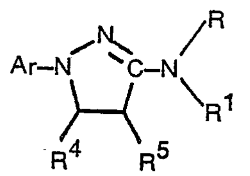

(I)

dadurch gekennzeichnet, daß

Ar eine Gruppe ist, die ausgewählt ist unter Phenyl, Naphthyl, Chinolyl und Pyridyl, wobei die Gruppe gegebenenfalls substituiert sein kann durch einen oder mehrere Substituenten, die ausgewählt sind unter Halogen, $C_{1-6}$-Alkyl (welches selbst durch ein oder mehrere Halogenatome substituiert sein kann), Carboxy, $C_{1-6}$-Alkoxy, Amino (welches selbst gegebenenfalls durch ein oder zwei $C_{1-6}$-Alkylreste substituiert sein kann), Hydroxy, und $C_{1-6}$-Alkylsulfonyl, worin die Alkyleinheit gegebenenfalls durch ein oder mehrere Halogenatome substituiert sein kann,

R ausgewählt ist unter Wasserstoff, $C_{1-6}$-Alkyl (welches gegebenenfalls durch einen Substituenten substituiert ist, der ausgewählt ist unter Phenyl, welches selbst in jeder beliebigen Position des Ringes substituiert sein kann durch einen oder mehrere Substituenten, die ausgewählt sind unter Halogen, $C_{1-6}$-Alkyl (welches selbst substituiert sein kann durch ein oder mehrere Halogenatome), Carboxy, $C_{1-6}$-Alkoxy, Amino (welches selbst gegebenenfalls substituiert sein kann durch ein oder zwei $C_{1-6}$-Alkylreste), Hydroxy, und $C_{1-6}$-Alkylsulfonyl, worin die Alkyleinheit gegebenenfalls substituiert sein kann durch ein oder mehrere Halogenatome, und Cycloalkyl mit 3 bis 6 Kohlenstoffatomen), $C_{2-6}$-Alkenyl und $C_{2-6}$-Alkinyl, $R^1$ ausgewählt ist unter $C_{1-6}$-Alkyl (welches gegebenenfalls substituiert ist durch einen substituenten, der ausgewählt ist unter Phenyl, welches selbst substituiert sein kann in irgendeiner Position des Rings durch einen oder mehrere Substituenten, welche ausgewählt sind unter Halogen, $C_{1-6}$-Alkyl (welches selbst substituiert sein kann durch ein oder mehrere Halogenatome), Carboxy, $C_{1-6}$-Alkoxy, Amino (welches selbst gegebenenfalls substituiert sein kann durch ein oder zwei $C_{1-6}$-Alkylreste), Hydroxy und $C_{1-6}$-Alkylsulfonyl, worin die Alkyleinheit gegebenenfalls substituiert sein kann durch ein oder mehrere Halogenatome, und Cycloalkyl mit 3 bis 6 Kohlenstoffatomen), $C_{2-6}$-Alkenyl und $C_{2-6}$-Alkinyl, und

$R^4$ und $R^5$ gleich oder unterschiedlich sind und jedes ausgewählt ist unter Wasserstoff und $C_{1-6}$-Alkyl, oder ein Säureadditionssalz davon, mit der Maßgabe, daß sofern Ar unsubstituiertes Phenyl ist und entweder (a) sofern R für Methyl steht, $R^1$ einen anderen Rest als Methyl darstellt oder (b) sofern R gleich Ethyl ist, $R^1$ einen anderen Rest als Wasserstoff und Ethyl darstellt.

13. Verbindung nach Anspruch 12, dadurch gekennzeichnet, daß sie der Formel ID entspricht

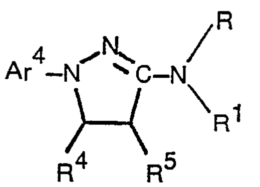

(ID)

worin

Ar ein monocyclischer aromatischer Rest ist, der ausgewählt ist unter Pyridyl, welches gegebenenfalls in einer beliebigen Position des Ringes durch einen oder mehrere Substituenten substituiert ist, und Phenyl, welches in einer beliebigen Position des Ringes durch einen oder mehrere Substituenten substituiert ist,

R ausgewählt ist unter Wasserstoff und $C_{1-6}$-Alkyl (welches gegebenenfalls durch Phenyl substituiert ist),

$R^1$ gleich $C_{1-6}$-Alkyl ist (welches gegebenenfalls durch Phenyl substituiert ist), und

$R^4$ und $R^5$ gleich oder unterschiedlich sind und jeweils unter Wasserstoff und $C_{1-6}$-Alkyl ausgewählt sind, und Säureadditionssalze davon.

14. 3-(Methylamino)-1-(3-trifluormethylphenyl)-2-pyrazolin und Säureadditionssalze davon.

15. Verfahren zur Herstellung einer Verbindung der Formel I gemäß der Definition in Anspruch 12, gekennzeichnet durch

(a) Cyclisierung und Eliminierung von Wasser aus einer Verbindung der Formel II

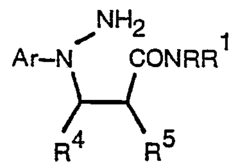

(II)

worin R, $R^1$, $R^4$, $R^5$ und Ar der Definition in Formel I entsprechen, oder durch

(b) Umsetzung einer Verbindung der Formel VII mit $R^8Y$

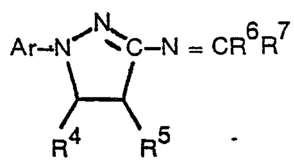

(VII)

worin Ar, $R^4$ und $R^5$ der Definition in Formel I entsprechen, Y ein Alkalimetall- oder ein Erdalkalimetallhalogenid darstellt, und $—CR^6R^7R^8$ gleich $R^1$ gemäß der Definition in Formel I ist, oder durch

(c) Reduktion einer Verbindung der Formel VII, worin Ar, $R^4$ und $R^5$ der Definition in Formel I entsprechen, und $—CHR^6R^7$ für $R^1$ steht gemäß der Definition in Formel I, oder durch

(d) Reduktion einer Verbindung der Formel X

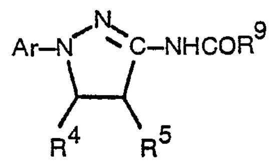

(X)

worin Ar, $R^4$ und $R^5$ der Definition in Formel I entsprechen, und entweder $CH_2R^9$ der Definition von $R^1$ in Formel I entspricht oder sofern $R^1$ gleich Methyl ist, $R^9$ Alkoxy sein kann,
und wahlweise Überführung eines so hergestellten Verbindung der Formel I in irgendeine andere Verbindung der Formel I.

**Revendications**

1. Formulation pharmaceutique comprenant un constituant actif en association avec un excipient pharmaceutiquement acceptable pour celui-ci, caractérisée en ce que le constituant actif est un composé de formule (I):

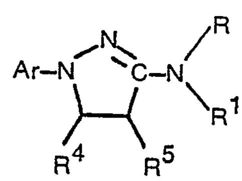

(I)

où

Ar représente un radical choisi parmi phényle, naphtyle, quinoléyle et pyridyle, lequel radical peut éventuellement être substitué par un ou plusieurs substituants choisis parmi halo, $C_{1-6}$ alcoyle (qui peut lui-même être substitué par un ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène;

R est choisi parmi hydrogène, $C_{1-6}$ alcoyle (éventuellement substitué par un substituant choisi parmi phényle qui peut lui-même être substitué en une position quelconque du cycle par un ou plusieurs substituants choisis parmi halo, $C_{1-6}$ alcoyle (qui peut lui-même être substitué par ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène, et cycloalcoyle de 3 à 6 atomes de carbone), $C_{2-6}$ alcényle et $C_{2-6}$ alcynyle;

$R^1$ est choisi parmi $C_{1-6}$ alcoyle (éventuellement substitué par un substituant choisi parmi phényle qui

peut lui-même être substitué en une position quelconque du cycle par un ou plusieurs substituants choisis parmi halo, $C_{1-6}$ alcoyle (qui peut lui-même être substitué par un ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène, et cycloalcoyle de 3 à 6 atomes de carbone), $C_{2-6}$ alcényle et $C_{2-6}$ alcynyle; et

$R^4$ et $R^5$ sont identiques ou différents et sont chacun choisis parmi hydrogène et $C_{1-6}$ alcoyle, ou un sel d'addition d'acide de ce composé.

2. Formulation suivant la revendication 1, caractérisée en ce que dans la formule (I), R est choisi parmi hydrogène, $C_{1-6}$ alcoyle (éventuellement substitué par un substituant choisi parmi phényle et cycloalcoyle de 3 à 6 atomes de carbone), $C_{2-6}$ alcényle et $C_{2-6}$ alcynyle, et $R^1$ est choisi parmi $C_{1-6}$ alcoyle (éventuellement substitué par un substituant choisi parmi phényle et cycloalcoyle de 3 à 6 atomes de carbone), $C_{2-6}$ alcényle et $C_{2-6}$ alcynyle.

3. Formulation suivant la revendication 1, caractérisée en ce que le constituant actif est choisi parmi
la 3-méthylamino-1-(3-trifluorométhylphényl)-2-pyrazoline;
la 3-éthylamino-1-(3-trifluorométhylphényl)-2-pyrazoline;
la 3-propylamino-1-(3-trifluorométhylphényl)-2-pyrazoline;
la 3-butylamino-1-(3-trifluorométhylphényl)-2-pyrazoline;
le chlorhydrate de 3-benzylamino-1-(3-trifluorométhylphényl)-2-pyrazoline;
la 3-(N,N-diméthylamino)-1-(4-trifluorométhylphényl)-2-pyrazoline;
la 3-(N-méthyl-N-éthylamino)-1-(4-chlorophényl)-2-pyrazoline;
la 3-(méthylamino)-1-(4-fluorophényl)-5-méthyl-2-pyrazoline;
la 3-(benzylamino)-1-(4-bromophényl)-4-méthyl-2-pyrazoline;
la 3-(éthylamino)-1-(3-trifluorométhyl-4-fluorophényl)-2-pyrazoline;
la 3-(t-butylamino)-1-(3-trifluorométhyl-4-bromophényl)-2-pyrazoline;
la 3-(isopropylamino)-1-(5-chloro-2-pyridyl)-2-pyrazoline;
la 3-(méthylamino)-1-(5-bromo-2-pyridyl)-2-pyrazoline;
la 3-(benzylamino)-1-(5-iodo-2-pyridyl)-2-pyrazoline;
la 3-(allylamino)-1-(3-trifluorométhylphényl)-2-pyrazoline;
la 3-(cyclohexylméthylamino)-1-(3-trifluorométhylphényl)-2-pyrazoline;
la 3-méthylamino-1-(5-méthyl-2-pyridyl)-2-pyrazoline;
la 3-méthylamino-1-(6-méthyl-2-pyridyl)-2-pyrazoline;
la 3-éthylamino-1-(6-méthyl-2-pyridyl)-2-pyrazoline;
la 3-méthylamino-1-(3-quinoléyl)-2-pyrazoline;
la 3-méthylamino-1-(4-chloro-3-trifluorométhylphényl)-2-pyrazoline;
la 3-méthylamino-1-(5-bromo-6-méthyl-2-pyridyl)-2-pyrazoline;
la 3-(N,N-diméthylamino)-1-(3-trifluorométhylphényl)-2-pyrazoline;
la 3-éthylamino-5-méthyl-1-phényl-2-pyrazoline;
la 3-butylamino-5-méthyl-1-phényl-2-pyrazoline;
la 3-(1-phényléthylamino)-1-(3-trifluorométhylphényl)-2-pyrazoline;
la 3-(2-buténylamino)-1-(2-naphtyl)-2-pyrazoline;
la 3-éthylamino-4-méthyl-1-(3-trifluorométhylphényl)-2-pyrazoline;
la 3-benzylamino-1-(3-t-butylphényl)-2-pyrazoline;
la 3-benzylamino-1-(3-carboxyphényl)-2-pyrazoline;
la 3-méthylamino-1-(2-chlorophényl)-2-pyrazoline;
la 3-benzylamino-1-(4-méthoxyphényl)-2-pyrazoline;
la 3-(4-chlorobenzylamino)-1-(4-chlorophényl)-2-pyrazoline et
la 3-(4-méthoxybenzylamino)-1-(4-bromo-3-trifluorométhylphényl)-2-pyrazoline.

4. Formulation pharmaceutique comprenant un constituant actif en association avec un excipient pharmaceutiquement acceptable pour celui-ci, caractérisée en ce que le constituant actif est la 3-(méthylamino)-1-(3-trifluorométhylphényl)-2-pyrazoline ou un sel d'addition d'acide pharmaceutiquement acceptable de celle-ci.

5. Formulation suivant l'une quelconque des revendications 1 à 4, caractérisée en ce qu'elle est présentée sous une forme se prêtant à l'administration ophtalmique.

6. Formulation suivant l'une quelconque des revendications 1 à 5, caractérisée en ce qu'elle est présentée sous la forme d'un collyre aqueux.

7. Formulation suivant l'une quelconque des revendications 1 à 6, caractérisée en ce que le constituant actif est en outre associé à un autre constituant thérapeutique choisi parmi les agents antibiotiques, les agents antifongiques et les agents antiviraux.

8. Composé de formule (I) tel que défini dans l'une quelconque des revendications 1 à 7, à utiliser pour la prophylaxie ou le traitement d'une affection médicale à laquelle de l'inflammation est associée.

9. Composé de formule (1) tel que défini dans l'une quelconque des revendications 1 à 7, à utiliser pour la prophylaxie ou le traitement d'une affection médicale choisie parmi les états arthritiques, les états inflammatoires de la peau, les états inflammatoires de l'oeil, la nécrose des tissus, la réjection de tissu après transplantation chirurgicale, la douleur, la pyrexie et l'asthme.

10. Composé de formule (I) tel que défini dans l'une quelconque des revendications 1 à 7, à utiliser en médecine pour l'inhibition des trajets du métabolisme de l'acide arachidonique par la lipoxygénase ou la cyclo-oxygénase.

11. La 3-méthylamino-1-(3-trifluorométhylphényl)-2-pyrazoline à utiliser pour la prophylaxie ou le traitement des états inflammatoires de l'oeil.

12. Composé de formule (I):

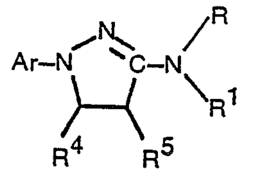

(I)

caractérisé en ce que

Ar représente un radical choisi parmi phényle, naphtyle, quinoléyle et pyridyle, lequel radical peut éventuellement être substitué par un ou plusieurs substituants choisis parmi halo, $C_{1-6}$ alcoyle (qui peut lui-même être substitué par un ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène;

R est choisi parmi hydrogène, $C_{1-6}$ alcoyle (éventuellement substitué par un substituant choisi parmi phényle qui peut lui-même être substitué en une position quelconque du cycle par un ou plusieurs substituants choisis parmi halo, $C_{1-6}$ alcoyle (qui peut lui-même être substitué par un ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène, et cycloalcoyle de 3 à 6 atomes de carbone), $C_{2-6}$ alcényle et $C_{2-6}$ alcynyle;

$R^1$ est choisi parmi $C_{1-6}$ alcoyle (éventuellement substitué par un substituant choisi parmi phényle qui peut lui-même être substitué en une position quelconque du cycle par un ou plusieurs substituants choisis parmi halo, $C_{1-6}$ alcoyle (qui peut lui-même être substitué par un ou plusieurs atomes d'halogène), carboxyle, $C_{1-6}$ alcoxy, amino (qui peut lui-même éventuellement être substitué par 1 ou 2 radicaux $C_{1-6}$ alcoyle), hydroxyle et $C_{1-6}$ alcoylsulfonyle dont l'entité alcoyle peut éventuellement être substituée par un ou plusieurs atomes d'halogène, et cycloalcoyle de 3 à 6 atomes de carbone), $C_{2-6}$ alcényle et $C_{2-6}$ alcynyle; et

$R^4$ et $R^5$ sont identiques ou différents et sont chacun choisis parmi hydrogène et $C_{1-6}$ alcoyle, ou sel d'addition d'acide de ce composé, avec la restriction que lorsque Ar est un radical phényle non substitué et que soit (a) lorsque R est un radical méthyle, alors $R^1$ est autre qu'un radical méthyle, soit (b) lorsque R est un radical éthyle, alors $R^1$ est autre qu'un atome d'hydrogène et qu'un radical éthyle.

13. Composé suivant la revendication 12, caractérisé en ce qu'il est de formule (ID):

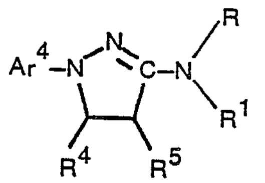

(ID)

où

$Ar^4$ est un radical aromatique monocyclique choisi parmi pyridyle qui est éventuellement substitué en une position quelconque du cycle par un plusieurs substituants et phényle substitué en une position quelconque du cycle par un ou plusieurs substituants;

R est choisi parmi hydrogène et $C_{1-6}$ alcoyle (éventuellement substitué par phényle);

$R^1$ est un radical $C_{1-6}$ alcoyle (éventuellement substitué par phényle); et

$R^4$ et $R^5$ sont identiques ou différents et sont chacun choisis parmi hydrogène et $C_{1-6}$ alcoyle; et les sels d'addition d'acides de ce composé.

14. La (3-méthylamino)-1-(3-trifluorométhylphényl)-2-pyrazoline et ses sels d'addition d'acides.

15. Procédé de préparation d'un composé de formule (I) tel que défini dans la revendication 12, caractérisé par

(a) la cyclisation et l'élimination d'eau d'un composé de formule (II):

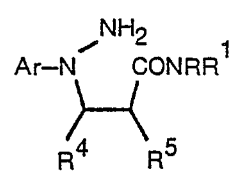

(II)

où R, $R^1$, $R^4$, $R^5$ et Ar sont tels que définis à propos de la formule (I), ou

(b) la réaction d'un composé de formule (VII) avec $R^8Y$:

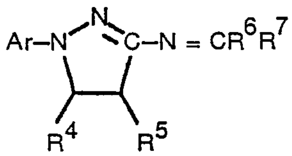

(VII)

où Ar, $R^4$ et $R^5$ sont tels que définis à propos de la formule (I), Y représente un métal alcalin ou halogénure de métal alcalino-terreux et $—CR^6R^7R^8$ représente $R^1$ tel que défini à propos de la formule (I), ou

(c) la réduction d'un composé de formule (VII) où Ar, $R^4$ et $R^5$ sont tels que définis à propos de la formule (I) et $—CH^6R^7$ représente $R^1$ tel que défini à propos de la formule (I), ou

(d) la réduction d'un composé de formule (X):

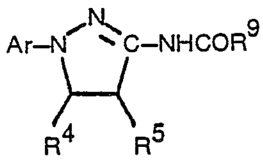

(X)

où Ar, $R^4$ et $R^5$ sont tels que définis à propos de la formule (I) et $CH_2R^9$ représente $R^1$ tel que défini à propos de la formule (I) ou, lorsque $R^1$ est un radical méthyle, $R^9$ peut être un radical alcoxy;
et éventuellement la conversion d'un composé quelconque de formule (I) ainsi obtenu en un autre composé de formule (I).